(19) Europäisches Patentamt
European Patent Office
Office européen des brevets

(11) **EP 3 532 595 B1**

(12) **EUROPEAN PATENT SPECIFICATION**

(45) Date of publication and mention
of the grant of the patent:
**21.01.2026 Bulletin 2026/04**

(21) Application number: **17809076.7**

(22) Date of filing: **27.10.2017**

(51) International Patent Classification (IPC):
**C11D 17/04** (2006.01)  **C08J 5/18** (2006.01)
**B65D 65/46** (2006.01)

(52) Cooperative Patent Classification (CPC):
**C08J 5/18; B65D 65/46; C11D 17/042;**
C08J 2329/04; C08J 2403/02; C08J 2403/08

(86) International application number:
**PCT/US2017/058657**

(87) International publication number:
**WO 2018/081494 (03.05.2018 Gazette 2018/18)**

(54) **WATER-SOLUBLE FILM WITH LOW COEFFICIENT OF FRICTION**

WASSERLÖSLICHE FOLIE MIT NIEDRIGEM REIBWERT

FILM SOLUBLE À L'EAU AVEC UN COEFFICIENT BAS DE FRICTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2016 US 201662413929 P**

(43) Date of publication of application:
**04.09.2019 Bulletin 2019/36**

(60) Divisional application:
**25214320.1 / 4 678 558**

(73) Proprietor: **MonoSol, LLC
Merrillville, Indiana 46410 (US)**

(72) Inventors:
• **LEE, David M.**
**Clemmons, NC 27012 (US)**
• **BROMBY, Percy, II**
**Merrillville, IN 46410 (US)**

(74) Representative: **Global IP Europe
Patentanwaltskanzlei
Pfarrstraße 14
80538 München (DE)**

(56) References cited:
EP-A1- 0 611 804    WO-A2-2004/074351
GB-A- 2 442 626    US-A1- 2016 280 869

• -: "MSTM-205: Standard Test Method for Solubility of MonoSol⬦ Water Soluble Film when contained within a Plastic Holder", 31 March 2003 (2003-03-31), XP55728684, Retrieved from the Internet <URL:https://www.monosol.com/wp-content/uploads/2017/01/CID604_MSTM-205_Water_soluble_film_contained_Plastic-Holder.pdf> [retrieved on 20200908]
• -: "MSTM-205: Standard Test Method for Solubility of MonoSol⬦ Water Soluble Film when contained within a Plastic Holder", 31 March 2003 (2003-03-31), XP055728684, Retrieved from the Internet <URL:https://www.monosol.com/wp-content/uploads/2017/01/CID604_MSTM-205_Water_soluble_film_contained_Plastic-Holder.pdf> [retrieved on 20200908]
• LAWTON J W: "Effect of starch type on the properties of starch containing films", CARBOHYDRATE POLYMERS, APPLIED SCIENCE PUBLISHERS, LTD. BARKING, GB, vol. 29, no. 3, 1 March 1996 (1996-03-01), pages 203 - 208, XP004069605, ISSN: 0144-8617, DOI: 10.1016/0144-8617(96)00028-8
• J A BORGES ET AL: "Influence of different starch sources and plasticizers on properties of biodegradable films", INTERNATIONAL FOOD RESEARCH JOURNAL, vol. 22, no. 6, 1 November 2015 (2015-11-01), Serdang, Selangor, pages 2346 - 2351, XP055484182, ISSN: 1985-4668

**(Cont. next page)**

- **SHI Y-C ET AL: "Molecular structure of a low-amylopectin starch and other high-amylose maize starches", JOURNAL OF CEREAL SCIENCE, ACADEMIC PRESS LTD, GB, vol. 27, no. 3, 1 May 1998 (1998-05-01), pages 289 - 299, XP002239696, ISSN: 0733-5210, DOI: 10.1006/ JCRS.1997.9998**

## Description

### Field of the Invention

The present disclosure relates generally to water-soluble films and related packets. More particularly the disclosure relates to polyvinyl alcohol based water-soluble films that can demonstrate low coefficients of friction, and thus a reduced tendency to stick to surfaces such as converting equipment and other water-soluble films.

### Background

Water-soluble polymeric films are commonly used as packaging materials to simplify dispersing, pouring, dissolving and dosing of a material to be delivered. A consumer can directly add the pouched composition to a mixing vessel, such as a bucket, sink or any vessel suitable for holding water. Advantageously, this provides for accurate dosing while eliminating the need for the consumer to measure the composition. The pouched composition may also reduce mess that would be associated with dispensing a material from a product container, such as pouring or scooping a material. In sum, soluble pre-measured polymeric film pouches provide for convenience of consumer use in a variety of applications.

Patent document WO 2004/074351 A2 discloses a water-soluble film for packaging a non-liquid product, wherein the film is made of a hydrolyzed copolymer of vinyl acetate and methyl acrylate in a range of from about 30 to about 95 percent by weight; modified starch in a range of from about 4 to about 30 percent by weight; plasticizer in a range of from about 5 to about 30 percent by weight; lubricant/release agent in a range of from about 0.0 to about 1.5 percent by weight; and surfactant in a range of from about 0.01 to about 1.5 percent by weight. Preferably, the modified starch comprises hydroxyethyl modified starch.

US 2016/280869 A1 describes a water-soluble film useful for packaging ingredients and releasing them into cold water after a desired amount of time. The film includes a mixture of a water-soluble polyvinyl alcohol and a plasticizer blend comprising glycerol, sorbitol, and 2-methyl-1,3-propanediol.

GB 2442626 A discloses a water-soluble film based on polyvinyl alcohol which exhibits a dissolution time in water at 20°C of not more than 60 sec in terms of a film thickness of 76 $\mu$m.

EP 0611804 A1 relates to melt extrudable polyvinyl alcohol compositions consisting essentially of polyvinyl alcohol and 1-30 wt% plasticizer which is useful for packaging and molding applications. The composition is prepared by melt blending polyvinyl alcohol and thermoplastic modified starch to provide a product which has reduced water sensitivity, improved modulus and reduced elongation at high relative humidity.

The publication "Effect of starch type on the properties of starch containing films" by Lawton J. W. (CARBOHYDRATE POLYMERS, APPLIED SCIENCE PUBLISHERS, LTD. BARKING, GB, vol. 29, no. 3, 1 March 1996 (1996-03-01), pages 203-208) refers to starch-poly(vinyl alcohol) cast films containing 41% starch, 41% PVA, 15% glycerol and 3% poly(ethylene-co-acrylic acid).

However, water-soluble films prepared from water-soluble polymers may stick to the surface on which they are formed (or converted into, e.g., pouches) and/or to other water-soluble films. Such problems may particularly arise when the film is formed into pouches and the pouches are stored together in secondary packaging. Anti-blocking agents have been used to reduce the tackiness of the water-soluble films in an effort to prevent adhesion to the manufacturing surface and pouch to pouch adhesion during storage of pouches. However, inclusion of anti-blocking agents, particularly at higher loading levels, generally has a detrimental effect on the mechanical properties of the resulting films. In particular, the tensile strength of the water-soluble films generally decreases, resulting in difficulty processing the films into single dose pouches. Alternative solutions include powdering the film with talc, starch, or similar particles; however, the powdering process is generally messy and results in wasted powder materials and machine down time.

Thus, there exists a need in the art for water-soluble films that can be formed into water-soluble packages and remain water-soluble with a reduced tendency to stick to other water-soluble films.

### Summary

One aspect of the disclosure provides a water-soluble film, including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film, and the hydroxypropyl-modified starch has an amylose content in a range of 65% to 95% by weight, based on the total weight of amylose and amylopectin in the hydroxypropyl-modified starch, and the hydroxypropyl-modified starch is present in an amount in a range of 5 phr to 30 phr, wherein phr denotes parts per one hundred parts of the total water-soluble polyvinyl alcohol content of the film.

A related aspect of the disclosure provides a sealed article including a water-soluble film in the form of a pouch defining an interior pouch volume, the water-soluble film including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film, and the hydroxypropyl-modified starch has an amylose content in a range of 65% to 95% by weight, based on the total weight of amylose and amylopectin in the hydroxypropyl-modified starch, and the hydroxypropyl-modified starch is present in an amount in a range of 5 phr to 30 phr, wherein phr denotes parts per one hundred parts of the total water-soluble polyvinyl alcohol content of the film.

Another aspect of the disclosure provides a water-soluble film, including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film and the hydroxypropyl-modified starch has a degree of modification of greater than 2% by weight, has an amylose content in a range of 65% to 95% by weight, based on the total weight of amylose and amylopectin in the hydroxypropyl-modified starch, and is present in an amount in a range of 2.5 phr to 30 phr, wherein phr denotes parts per one hundred parts of the total water-soluble polyvinyl alcohol content of the film.

A related aspect of the disclosure provides a sealed article including a water-soluble film in the form of a pouch defining an interior pouch volume, the water-soluble film including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film and the hydroxypropyl-modified starch has a degree of modification of greater than 2% by weight, has an amylose content in a range of 65% to 95% by weight, based on the total weight of amylose and amylopectin in the hydroxypropyl-modified starch, and is present in an amount in a range of 2.5 phr to 30 phr, wherein phr denotes parts per one hundred parts of the total water-soluble polyvinyl alcohol content of the film.

Another aspect of the disclosure provides a water-soluble film, including a mixture of a water-soluble polyvinyl alcohol with a plasticizer and a hydroxypropyl-modified starch, wherein the polyvinyl alcohol is present in an amount of 20 wt.% to 95 wt.%, based on the total weight of the film, the starch has an amylose content in a range of 23% to 95% by weight based on the total weight of amylose and amylopectin in the hydroxypropyl-modified starch, and is present in an amount in a range of 5 phr to 30 phr, wherein phr denotes parts per one hundred parts of the total water-soluble polyvinyl alcohol content of the film and the polyvinyl alcohol comprises an unmodified polyvinyl alcohol or an anionic modified polyvinyl alcohol copolymer with the proviso that the anionic modifier is not an acrylate.

A related aspect of the disclosure provides a sealed article including a water-soluble film in the form of a pouch defining an interior pouch volume, the water-soluble film including a mixture of a water-soluble polyvinyl alcohol with a plasticizer and a hydroxypropyl-modified starch, wherein the polyvinyl alcohol is present in an amount of 20 wt.% to 95 wt.%, based on the total weight of the film, the starch has an amylose content in a range of 23% to 95% by weight based on the total weight of amylose and amylopectin in the hydroxypropyl-modified starch, and is present in an amount in a range of 5 phr to 30 phr, wherein phr denotes parts per one hundred parts of the total water-soluble polyvinyl alcohol content of the film and the polyvinyl alcohol comprises an unmodified polyvinyl alcohol or an anionic modified polyvinyl alcohol copolymer with the proviso that the anionic modifier is not an acrylate.

Another aspect of the disclosure provides a sealed article including a water-soluble film in the form of a pouch defining an interior pouch volume, the water-soluble film being defined according to any aspect or embodiment described herein.

For the compositions described herein, optional features, including but not limited to components and compositional ranges thereof, are contemplated to be selected from the various aspects, embodiments, and examples provided herein. For example, the embodiments and formulation approaches described in Examples 6 to 45 can be combined with any of the additional features provided in the description herein.

## Brief Description of the Drawings

For further facilitating the understanding of the present invention two drawing figures are appended hereto.

Figure 1 shows a rendering of amylose and amylopectin.
Figure 2 shows an example of an apparatus for measuring the coefficient of friction of a film specimen.

## Detailed Description

One aspect of the disclosure provides a water-soluble film, including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film, and the hydroxypropyl-modified starch has an amylose content in a range of 65% to 95% by weight, based on the total weight of amylose and amylopectin in the hydroxypropyl-modified starch, and the hydroxypropyl-modified starch is present in an amount in a range of 5 phr to 30 phr.

The films according to this aspect surprisingly may be designed to provide a combination of (1) excellent ability to be converted into a pouch using automated equipment at high starch loadings (convertibility) as characterized by a tensile strength in a range of 45 MPa to 60 MPa according to the Tensile Strength Test described below; (2) excellent water solubility as characterized by a cold water (10°C) dissolution time of 100 seconds or less according to MSTM-205; and (3) good film-to-film anti-stick properties as characterized by a relatively low gloss-to-gloss static coefficient of friction (COF) of 5 or less, as measured by the Coefficient of Friction Test described below.

A related aspect of the disclosure provides a sealed article including a water-soluble film in the form of a pouch defining an interior pouch volume, the water-soluble film including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film, and the hydroxypropyl-modified starch has an amylose content in a range of 65% to 95% by weight, based on the total weight of amylose and amylopectin in the hydroxypropyl-modified starch, and the hydroxypropyl-modified starch is present in an amount in a range of 5 phr to 30 phr.

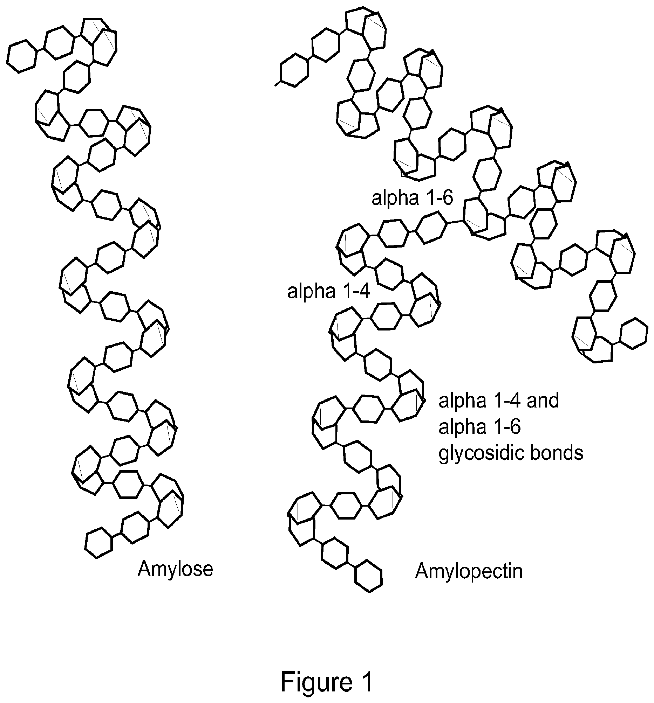

The hydroxypropylated starch has an amylose content in a range of 65% to 95%. As used herein, and unless specified otherwise, the percentage of amylose is a weight percentage based on the total weight of amylose and amylopectin in a starch consisting essentially of water, amylose, and amylopectin having about 12 wt.% water. For example, a starch having a 65% amylose content has an amylopectin content of 35%. As shown in Figure 1, amylose comprises a linear helical polymer of $\alpha$-D-glucose units linked through $\alpha(1{\to}4)$ glycosidic bonds and amylopectin comprises highly branched polymers of glucose linked in a linear way with (1-4) glycosidic bonds and branching taking place with $\alpha(1{\to}6)$ bonds, e.g., occurring every 24 to 30 glucose units. Without intending to be bound by theory, it is believed that typical modified starches (e.g., hydroxypropylated, hydroxyethylated starches, or acetate modified starches) have modifications (e.g., hydroxypropyl, hydroxyethyl, or acetate groups) distributed randomly or substantially randomly throughout both the amylose and amylopectin chains. In one aspect, the modified starch (e.g., hydroxypropylated or hydroxyethylated starch) for use herein will have modifications distributed randomly throughout both the amylose and amylopectin chains. In an alternative aspect, the modified starch (e.g., hydroxypropylated or hydroxyethylated starch) for use herein will have modifications distributed non-randomly throughout both the amylose and amylopectin chains.

In embodiments, the hydroxypropylated starch may have a degree of modification in a range of 4% to 8%. As used herein, and unless specified otherwise, the degree of modification of a starch is a weight percent. The modification of a starch can also be characterized as a degree of substitution (DS), between 0 and 3, wherein a DS of 0 means there are no modified OH groups on each glucose ring and a DS of 3 means that all 3 OH groups on each glucose ring have been modified. In embodiments, the hydroxypropylated starch may have a DS in a range of about 0.05 to about 0.4, or about 0.1 to about 0.3, or about 0.1, about 0.2, about 0.3, or about 0.4, for example. In embodiments, the starch is present in an amount in a range of 6 phr to 16 phr. In embodiments, the starch is present in an amount in a range of 6 phr to 10 phr. In embodiments, the starch is present in the water-soluble film in an amount in a range of 12 phr to 16 phr. In embodiments, the water-soluble film has a tensile strength of at least 40 MPa as determined by the Tensile Strength Test described herein. In embodiments, the polyvinyl alcohol comprises a polyvinyl alcohol selected from the group consisting of a polyvinyl alcohol homopolymer, a polyvinyl alcohol copolymer having an anionic modification, and combinations of the foregoing.

In embodiments, the water-soluble film has a gloss to gloss (G-G) static coefficient of friction of 5 or less, or 0.1 to 5, or 0.5 to 5, as determined by the Coefficient of Friction Test described herein. The gloss side of a water-soluble film refers to the air side of a water-soluble film cast onto a casting surface. The matte side of a water-soluble film refers to casting surface side of a water-soluble film cast onto a casting surface. In embodiments, the water-soluble film is characterized by a reduced G-G static coefficient of friction compared to the equivalent film formulation that does not include a starch. The lower the G-G static coefficient of friction, the less likely the film (or pouch formed therefrom) is to stick to the surface on which it is formed (or converted into, e.g., pouches) and/or to other water-soluble films. As used herein, "gloss to gloss static coefficient of friction" refers to the static coefficient of friction between the gloss sides of two water-soluble films having the same formulation. As used herein, "gloss to matte static coefficient of friction" refers to the static coefficient of friction between a gloss side and a matte side of two water-soluble films having the same formulation. As used herein, "matte to matte static coefficient of friction" refers to the static coefficient of friction between the matte sides of two water-soluble films having the same formulation. The coefficients of frictions described herein are gloss-to-gloss static coefficient of frictions. The gloss-to-gloss static coefficient of friction is typically higher than the gloss-to-matte static coefficient of friction and the matte-to-matte coefficient of friction for a given cast water-soluble film. Without intending to be bound by theory, it is believed that the gloss-to-gloss static coefficient of friction of a cast film is representative of the static coefficient of friction of a blown film having the same film formulation as the cast film, as blown films are not cast onto a casting surface and, therefore, all sides of a blown film can be considered air or "gloss" sides.

In embodiments, the sealed article comprises a composition contained in the interior pouch volume. In embodiments the composition contained in the interior pouch volume is a detergent composition. In embodiments, the detergent composition comprises a surfactant, a bleach, an enzyme, a perfume, a dye or colorant, a solvent and combinations thereof.

Another aspect of the disclosure provides a water-soluble film, including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film and the modified starch has a degree of modification of greater than 2% by weight, has an amylose content in a range of 65% to 95% by weight, based on the total weight of amylose and amylopectin in the hydroxypropyl-modified starch, and the hydroxypropyl-modified starch is present in an amount in a range of 2.5 phr to 30 phr.

The films according to this aspect surprisingly may be designed to provide a combination of (1) excellent ability to be converted into a pouch using automated equipment at high starch loadings (convertibility) as characterized by a tensile strength in a range of 45 MPa to 60 MPa according to the Tensile Strength Test described below; (2) excellent water solubility as characterized by a cold water (10°C) dissolution time of 100 seconds or less according to MSTM-205; and (3) good film-to-film anti-stick properties as characterized by a relatively low gloss-to-gloss static coefficient of friction (COF) of 5 or less, as measured by the Coefficient of Friction Test described below.

A related aspect of the disclosure provides a sealed article including a water-soluble film in the form of a pouch defining an interior pouch volume, the water-soluble film including a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film and the modified starch has a degree of modification of greater than 2% by weight, has an amylose content in a range of 65% to 95% by weight, based on the total weight of amylose and amylopectin in the hydroxypropyl-modified starch, and the hydroxypropyl-modified is present in an amount in a range of 2.5 phr to 30 phr.

In embodiments, the modified starch has a degree of modification of 4% to 8%. In embodiments, the modified starch has an amylose content in a range of 65% to 95%. The starch is hydroxypropyl-modified. As used herein, "alkyl" refers to straight chained and branched saturated hydrocarbon groups containing one to thirty carbon atoms, for example, one to twenty carbon atoms, or one to ten carbon atoms. Nonlimiting examples of alkyl groups include, methyl, ethyl, n-propyl, isopropyl, n-butyl, sec-butyl (2-methylpropyl), t-butyl (1,1-dimethylethyl), 3,3-dimethylpentyl, and 2-ethylhexyl. Unless otherwise indicated, an alkyl group can be an unsubstituted alkyl group or a substituted alkyl group. As used herein, "hydroxyalkyl" refers to an alkyl group that is substituted with a hydroxyl group. Nonlimiting examples of hydroxyalkyl groups include hydroxymethyl, hydroxyethyl, hydroxypropyl, hydroxybutyl and the like.

In embodiments, the polyvinyl alcohol comprises a polyvinyl alcohol selected from the group consisting of a polyvinyl alcohol homopolymer, a polyvinyl alcohol copolymer having an anionic modification, and combinations of the foregoing. In embodiments, starch is present in an amount in a range of 6 phr to 16 phr. In embodiments, starch is present in an amount in a range of 6 phr to 10 phr. In embodiments, starch is present in an amount in a range of 12 phr to 16 phr. In embodiments, the water-soluble film has a tensile strength of at least 40 MPa and up to 60 MPa, as determined by the Tensile Strength Test described herein. In embodiments, the water-soluble film has a G-G static coefficient of friction of 5 or less, or 0.1 to 5, or 0.5 to 5, or 0.1 to 3, or 0.1 to 2, as determined by the Coefficient of Friction Test described herein. In embodiments, the water-soluble film is characterized by a reduced G-G static coefficient of friction compared to the equivalent film formulation that does not include a starch.

In embodiments, the sealed article comprises a composition contained in the interior pouch volume. In embodiments the composition contained in the interior pouch volume is a detergent composition. In embodiments, the detergent composition comprises a surfactant, a bleach, an enzyme, a perfume, a dye or colorant, a solvent and combinations thereof.

Another aspect of the disclosure provides a water-soluble film, including a mixture of a water-soluble polyvinyl alcohol with a plasticizer and a hydroxypropyl-modified starch, wherein the polyvinyl alcohol is present in an amount of 20 wt.% to 95 wt.%, based on the total weight of the film, the starch has an amylose content in a range of 23% to 95% by weight, based on the total weight of amylose and amylopectin in the hydroxypropyl-modified starch, and the hydroxypropyl-modified is present in an amount in a range of 5 phr to 30 phr and the polyvinyl alcohol comprises an unmodified polyvinyl alcohol or an anionic modified polyvinyl alcohol copolymer with the proviso that the anionic modifier is not an acrylate.

The films according to this aspect surprisingly be designed to provide a combination of (1) excellent ability to be converted into a pouch using automated equipment at high starch loadings (convertibility) as characterized by a tensile strength in a range of 45 MPa to 60 MPa according to the Tensile Strength Test described below; (2) excellent water solubility as characterized by a cold water (10°C) dissolution time of 100 seconds or less; and (3) good film-to-film anti-stick properties as characterized by a relatively low gloss-to-gloss static coefficient of friction (COF) of 5 or less, as measured by the Coefficient of Friction Test described below.

A related aspect of the disclosure provides a sealed article including a water-soluble film in the form of a pouch defining an interior pouch volume, the water-soluble film including a mixture of a water-soluble polyvinyl alcohol with a plasticizer and a hydroxypropyl-modified starch, wherein the polyvinyl alcohol is present in an amount of 20 wt.% to 95 wt.%, based on the total weight of the film, the starch has an amylose content in a range of 23% to 95% by weight, based on the total weight of amylose and amylopectin in the hydroxypropyl-modified starch, and the hydroxypropyl-modified starch is present in an amount in a range of 5 phr to 30 phr and the polyvinyl alcohol comprises an unmodified polyvinyl alcohol or an anionic modified polyvinyl alcohol copolymer with the proviso that the anionic modifier is not an acrylate or an alkyl acrylate.

In embodiments, the starch is present in an amount in a range of 5 phr to 12 phr. In embodiments, the water-soluble film has a G-G static coefficient of friction of 5 or less, or 0.1 to 5, or 0.5 to 5, or 0.1 to 3, or 0.1 to 2. In embodiments, the starch has an amylose content in a range of 50 wt.% to 95 wt.%. In embodiments, the hydroxypropyl modified starch includes greater than 2% hydroxypropyl modification, e.g. 2.1% to 8% hydroxypropyl modification. In embodiments, the hydroxypropyl modified starch includes from 4% to 8% hydroxypropyl modification. In embodiments, the water-soluble film is characterized by a reduced gloss to gloss static coefficient of friction compared to the equivalent film formulation that does not include a starch.

In embodiments, the sealed article comprises a composition contained in the interior pouch volume. In embodiments the composition contained in the interior pouch volume is a detergent composition. In embodiments, the detergent composition comprises a surfactant, a bleach, an enzyme, a perfume, a dye or colorant, a solvent and combinations thereof.

To be considered a water-soluble film according to the present disclosure, the film, at a thickness of about 1.5 mil (about 0.038 mm), dissolves in 300 seconds or less in water at a temperature of 20 °C (68 °F) in accordance with MonoSol Test Method MSTM-205.

"Comprising" as used herein means that various components, ingredients or steps that can be conjointly employed in practicing the present disclosure. Accordingly, the term "comprising" encompasses the more restrictive terms "consisting essentially of" and "consisting of." The present compositions can comprise, consist essentially of, or consist of any of the required and optional elements disclosed herein. For example, a thermoformed packet can "consist essentially of" a film described herein for use of it thermoforming characteristics, while including a non-thermoformed film (e.g., lid portion), and optional markings on the film, e.g. by inkjet printing. The invention illustratively disclosed herein suitably may be practiced in the absence of any element or step which is not specifically disclosed herein.

All percentages, parts and ratios referred to herein are based upon the total dry weight of the film composition or total weight of the packet content composition of the present disclosure, as the case may be, and all measurements made are at about 25°C, unless otherwise specified. All such weights as they pertain to listed ingredients are based on the active level and therefore do not include carriers or by-products that may be included in commercially available materials, unless otherwise specified.

All ranges set forth herein include all possible subsets of ranges and any combinations of such subset ranges. By default, ranges are inclusive of the stated endpoints, unless stated otherwise. Where a range of values is provided, it is understood that each intervening value between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also contemplated to be part of the disclosure.

It is expressly contemplated that for any number value described herein, e.g. as a parameter of the subject matter described or part of a range associated with the subject matter described, an alternative which forms part of the description is a functionally equivalent range surrounding the specific numerical value (e.g. for a dimension disclosed as "40 mm" an alternative embodiment contemplated is "about 40 mm"). Likewise, a value described by "about" expressly includes as an alternative embodiment the specific value itself (e.g. for an endpoint described as "about 40" an alternative embodiment contemplated is "40").

As used herein, the terms packet(s) and pouch(es) should be considered interchangeable. In certain embodiments, the terms packet(s) and pouch(es), respectively, are used to refer to a container made using the film and a sealed container preferably having a material sealed therein, e.g., in the form a measured dose delivery system. The sealed pouches can be made from any suitable method, including such processes and features such as heat sealing, solvent welding, and adhesive sealing (e.g., with use of a water-soluble adhesive).

As used herein and unless specified otherwise, the terms "wt.%" and "wt%" are intended to refer to the composition of the identified element in "dry" (non water) parts by weight of the entire film, including residual moisture in the film (when applicable) or parts by weight of the entire composition enclosed within a pouch (when applicable).

As used herein and unless specified otherwise, the term "PHR"("phr") is intended to refer to the composition of the identified element in parts per one hundred parts water-soluble polymer resin (whether PVOH or other polymer resin, but not including starch) in the water-soluble film.

The film can be made by any suitable method, including a solution casting method. The film can be used to form a container (pouch) by any suitable process, including vertical form, fill, and sealing (VFFS), rotary drum horizontal form, fill, and sealing, or thermoforming and, for example, solvent sealing or heat sealing of film layers around a periphery of the container. The pouches can be used for dosing materials to be delivered into bulk water, for example.

In any embodiment, the water-soluble pouch can contain (enclose) a composition in the defined interior volume of the pouch. The composition can be selected from a liquid, solid or combination thereof. As used herein, "liquid" includes free-flowing liquids, as well as pastes, gels, foams and mousses. Non-limiting examples of liquids include detergent compositions such as liquid light duty and liquid heavy duty liquid detergent compositions, powdered detergent compositions, dish detergent for hand washing and/or machine washing; hard surface cleaning compositions, fabric enhancers, detergent gels commonly used for laundry, and bleach and laundry additives, shaving creams, skin care, hair care compositions (shampoos and conditioners), and body washes. Such detergent compositions may comprise a surfactant, a bleach, an enzyme, a perfume, a dye or colorant, a solvent and combinations thereof. Optionally, the detergent composition is selected from the group consisting of a laundry detergent, a dishwashing detergent, a hard surface cleaning composition, a fabric enhancer composition, a shampoo, a body wash, and combinations thereof.

Further non-limiting examples of liquids include non-household care compositions include agricultural compositions, automotive compositions, aviation compositions, food and nutritive compositions, industrial compositions, livestock compositions, marine compositions, medical compositions, mercantile compositions, military and quasi-military compositions, office compositions, and recreational and park compositions, pet compositions, water-treatment compositions, including cleaning and detergent compositions applicable to any such use. In embodiments, the composition contained in the interior pouch volume is a detergent composition.

Gases, e.g., suspended bubbles, or solids, e.g. particles, may be included within the liquids. A "solid" as used herein includes, but is not limited to, powders, agglomerates, and mixtures thereof. Non-limiting examples of solids include: granules, micro-capsules, beads, noodles, and pearlized balls. Solid compositions may provide a technical benefit including, but not limited to, through-the-wash benefits, pre-treatment benefits, and/or aesthetic effects such as microcapsules with perfumes or microcapsules with enzymes.

The film, pouches, and related methods of making and use are contemplated to include embodiments including any combination of one or more of the additional optional elements, features, and steps further described below (including those shown in the Examples), unless stated otherwise.

## WATER-SOLUBLE FILM

The water-soluble film described herein comprises a polyvinyl alcohol (PVOH) resin, a modified polyvinyl alcohol resin, or combinations thereof. In embodiments, the PVOH includes a PVOH resin selected from the group consisting of a PVOH homopolymer, a PVOH copolymer having an anionic modification, and combinations of the foregoing. The film can have any suitable thickness, and a film thickness of about 76 microns ($\mu$m) is typical and particularly contemplated. Other values and ranges contemplated include values in a range of about 5 to about 200 $\mu$m, or in a range of about 20 to about 100 $\mu$m, or about 40 to about 90 $\mu$m, or about 50 to 80 $\mu$m, or about or about 60 to 65 $\mu$m for example 65 $\mu$m, 76 $\mu$m, or 88 $\mu$m.

## PVOH RESIN

The film described herein includes one or more PVOH polymers to make up the PVOH resin content of the film, and can include a PVOH copolymer.

Polyvinyl alcohol is a synthetic resin generally prepared by the alcoholysis, usually termed hydrolysis or saponification, of polyvinyl acetate. Fully hydrolyzed PVOH, where virtually all the acetate groups have been converted to alcohol groups, is a strongly hydrogen-bonded, highly crystalline polymer which dissolves only in hot water - greater than about 140 °F (about 60 °C). If a sufficient number of acetate groups are allowed to remain after the hydrolysis of polyvinyl acetate, that is the PVOH polymer is partially hydrolyzed, then the polymer is more weakly hydrogen-bonded, less crystalline, and is generally soluble in cold water -- less than about 50 °F (about 10 °C). As such, the partially hydrolyzed polymer is a vinyl alcohol-vinyl acetate copolymer that is a PVOH copolymer, but is commonly referred to as PVOH.

As used herein, the term "homopolymer" generally includes polymers having a single type of monomeric repeating unit (e.g., a polymeric chain consisting of or consisting essentially of a single monomeric repeating unit). For the particular case of PVOH, the term "homopolymer" (or "PVOH homopolymer" or "PVOH polymer") further includes copolymers having a distribution of vinyl alcohol monomer units and vinyl acetate monomer units, depending on the degree of hydrolysis (e.g., a polymeric chain consisting of or consisting essentially of vinyl alcohol and vinyl acetate monomer units). In the limiting case of 100% hydrolysis, a PVOH homopolymer can include a true homopolymer having only vinyl alcohol units.

The viscosity of a PVOH polymer ($\mu$) is determined by measuring a freshly made solution using a Brookfield LV type viscometer with UL adapter as described in British Standard EN ISO 15023-2:2006 Annex E Brookfield Test method. It is international practice to state the viscosity of 4% aqueous polyvinyl alcohol solutions at 20 °C. All viscosities specified herein in Centipoise (cP) should be understood to refer to the viscosity of 4% aqueous polyvinyl alcohol solution at 20 °C, unless specified otherwise. Similarly, when a resin is described as having (or not having) a particular viscosity, unless specified otherwise, it is intended that the specified viscosity is the average viscosity for the resin, which inherently has a corresponding molecular weight distribution.

When the PVOH resin is a PVOH polymer blend, the resin can be selected based upon the weighted log average viscosity ($\overline{\mu}$). The $\overline{\mu}$ for a PVOH resin that comprises two or more PVOH polymers is calculated by the formula $\overline{\mu} = e^{\Sigma W_i \cdot \ln \overline{\mu_i}}$ where $\mu_t$ is the viscosity for the respective PVOH polymers.

For reference, the water-soluble film includes a PVOH resin or resin blend having a 4% solution viscosity at 20°C ($\overline{\mu}$). In various embodiments, the viscosity $\overline{\mu}$ can be in a range of about 4 cP to about 30 cP, about 5 cP to about 28 cP, about 12 cP to about 25 cP, about 18 cP to about 26 CP, about 14 cP to about 23 cP, about 22 cP to about 26 cP, or about 15 cP to about 20 cP, for example, about 10 cP, about 11 cP, about 12 cP, about 13 cP, about 14cP, about 14.5 cP, about 15 cP, about 16 cP, about 16.5 cP, about 17 cP, about 17.5 cP, about 18 cP, about 18.5 cP, about 19 cP, about 19.5 cP, about 20 cP, about 21 cP, about 22 cP, about 23 cP, about 24 cP, about 25 cP, about 26 cP, about 27 cP, about 28 cP, about 29 cP, or about 30 cP. It is well known in the art that the viscosity of PVOH resins is correlated with the weight average molecular weight ($\overline{M}w$) of the PVOH resin, and often the viscosity is used as a proxy for the Mw.

The PVOH resins can have a degree of hydrolysis (D.H. or DH) of at least 80%, 84% or 85% and at most about 99.7%, 98%, 96%, or 80%, for example in a range of about 84% to about 90%, or 85% to 88%, or 86.5%, or in a range of 85% to 99.7 %, about 88% to 98%, or 90% to 96%, for example 90%, 91%, 92%, 93%, 94%, 95%, or 96%, or in a range of about 87 to 93. As used herein, the degree of hydrolysis is expressed as a mole percentage of vinyl acetate units converted to vinyl alcohol units..

Furthermore, when the PVOH resin is a PVOH polymer blend, it is desirable to choose a PVOH blend that has an arithmetic weighted, average degree of hydrolysis ($\overline{H}°$ ) between about 80% and about 99.7%, between about 85 and about 98 %, or between about 87 and about 93 %, or about 96 and 98%, for example, about 85%, or about 86%, or about 87%, or about 87.5%, or about 88% or about 88.5%, or about 89%, or about 89.5%, or about 90%, or about 91%, or about 92%, or about 93%, or about 94%, or about 95%, or about 96%, or about 96.5%, or about 97%, or about 97.5%, or about 98%. For example, $\overline{H}°$ for a PVOH resin that comprises two or more PVOH polymers is calculated by the formula $\overline{H}° = \Sigma(W_i \cdot H_i)$ where $W_i$ is the weight percentage of the respective PVOH polymer and $H_i$ is the respective degrees of hydrolysis.

The water-soluble films can include PVOH copolymers which can be a PVOH terpolymer including vinyl alcohol monomer units, vinyl acetate monomer units (i.e., when not completely hydrolyzed), and a single type of anionic monomer unit (e.g., where a single type of monomer unit can include equivalent acid forms, salt forms, and optionally ester forms of the anionic monomer unit). In some aspects, the PVOH copolymer can include two or more types of anionic monomer units. General classes of anionic monomer units which can be used for the PVOH copolymer include the vinyl polymerization units corresponding to monocarboxylic acid vinyl monomers, their esters and anhydrides, dicarboxylic monomers having a polymerizable double bond, their esters and anhydrides, and alkali metal salts of any of the foregoing. Examples of suitable anionic monomer units include the vinyl polymerization units corresponding to vinyl anionic monomers including vinyl acetic acid, maleic acid, monoalkyl maleate, dialkyl maleate, maleic anhydride, fumaric acid, monoalkyl fumarate, dialkyl fumarate, fumaric anhydride, itaconic acid, monoalkyl itaconate, dialkyl itaconate, itaconic anhydride, citraconic acid, monoalkyl citraconate, dialkyl citraconate, citraconic anhydride, mesaconic acid, monoalkyl mesaconate, dialkyl mesaconate, mesaconic anhydride, glutaconic acid, monoalkyl glutaconate, dialkyl glutaconate, glutaconic anhydride, alkyl acrylates, alkyl alkacrylates, vinyl sulfonic acids, alkali metal salts of the foregoing, esters of the foregoing, and combinations of the foregoing. In embodiments, the anionic monomer unit is not an acrylate or an alkyl alkacrylate.

In embodiments, the anionic monomer unit is selected from the group consisting of vinyl acetic acid, alkyl acrylates, maleic acid, monoalkyl maleate, dialkyl maleate, monomethyl maleate, dimethyl maleate, maleic anhydride, fumaric acid, monoalkyl fumarate, dialkyl fumarate, monomethyl fumarate, dimethyl fumarate, fumaric anhydride, itaconic acid, monomethyl itaconate, dimethyl itaconate, itaconic anhydride, citraconic acid, monoalkyl citraconate, dialkyl citraconate, citraconic anhydride, mesaconic acid, monoalkyl mesaconate, dialkyl mesaconate, mesaconic anhydride, glutaconic acid, monoalkyl glutaconate, dialkyl glutaconate, glutaconic anhydride, vinyl sulfonic acid, alkyl sulfonic acid, ethylene sulfonic acid, 2-acrylamido-1-methyl propane sulfonic acid, 2-acrylamide-2-methylpropanesulfonic acid, 2-methylacrylamido-2-methylpropanesulfonic acid, 2-sulfoethyl acrylate, alkali metal salts of the foregoing, esters of the foregoing, and combinations of the foregoing. In embodiments, the anionic monomer unit is selected from the group consisting of vinyl acetic acid, maleic acid, monoalkyl maleate, dialkyl maleate, monomethyl maleate, dimethyl maleate, maleic anhydride, fumaric acid, monoalkyl fumarate, dialkyl fumarate, monomethyl fumarate, dimethyl fumarate, fumaric anhydride, itaconic acid, monomethyl itaconate, dimethyl itaconate, itaconic anhydride, citraconic acid, monoalkyl citraconate, dialkyl citraconate, citraconic anhydride, mesaconic acid, monoalkyl mesaconate, dialkyl mesaconate, mesaconic anhydride, glutaconic acid, monoalkyl glutaconate, dialkyl glutaconate, glutaconic anhydride, vinyl sulfonic acid, alkyl sulfonic acid, ethylene sulfonic acid, 2-acrylamido-1-methyl propane sulfonic acid, 2-acrylamide-2-methylpropanesulfonic acid, 2-methylacrylamido-2-methylpropanesulfonic acid, alkali metal salts of the foregoing, esters of the foregoing, and combinations of the foregoing. In embodiments, the anionic monomer unit is selected from the group consisting of maleic acid, monoalkyl maleate, dialkyl maleate, monomethyl maleate, dimethyl maleate, maleic anhydride, fumaric acid, monoalkyl fumarate, dialkyl fumarate, monomethyl fumarate, dimethyl fumarate, fumaric anhydride, itaconic acid, monomethyl itaconate, dimethyl itaconate, itaconic anhydride, citraconic acid, monoalkyl citraconate, dialkyl citraconate, citraconic anhydride, mesaconic acid, monoalkyl mesaconate, dialkyl mesaconate, mesaconic anhydride, glutaconic acid, monoalkyl glutaconate, dialkyl glutaconate, glutaconic anhydride, alkali metal salts of the foregoing, esters of the foregoing, and combinations of the foregoing. In embodiments, the anionic monomer unit is not an acrylate or an alkyl alkacrylate.

In one type of embodiment, the PVOH is a carboxyl group modified copolymer. In another aspect, the PVOH can be modified with a dicarboxyl type monomer. In one class of these embodiments, the $\alpha$ carbon of both carbonyls are connected to the unsaturated bond (e.g., maleic acid, fumaric acid). In another class of these embodiments, the $\alpha$ carbon of both carbonyls are connected to the unsaturated bond and the unsaturated bond is further substituted, e.g., with a methyl branch (e.g., citraconic acid, mesaconic acid). In another class of these embodiments, the $\beta$ carbon of one carbonyl and the $\alpha$ carbon of the other carbonyl are connected to the unsaturated bond (e.g., itaconic acid, cis-glutaconic acid, trans-glutaconic acid). Monomers that provide alkyl carboxyl groups are contemplated. A maleic acid type (e.g., maleic acid, dialkyl maleate (including dimethyl maleate), monoalkyl maleate (including monomethyl maleate), or maleic anhydride) comonomer is particularly contemplated.

In embodiments, the anionic monomer unit comprises a maleic acid derived monomer. The maleic acid derived monomer can be one or more of maleic acid, monoalkyl maleate, dialkyl maleate, monomethyl maleate, dimethyl maleate, maleic anhydride, alkali metal salts of the foregoing (e.g., sodium, potassium, or other alkali metal salts), esters of the foregoing (e.g., methyl, ethyl, or other $C_1$-$C_4$ or $C_6$ alkyl esters), and combinations thereof (e.g., multiple types of anionic monomers or equivalent forms of the same anionic monomer). For example, the maleic acid derived monomer can include one or more monoalkyl maleate, dialkyl maleate, and alkali metal salts thereof (e.g., sodium salts). Similarly, the anionic monomer can include one or more of monomethyl maleate, dimethyl maleate and alkali metal salts thereof (e.g., sodium salts).

The solvent for the saponification of polyvinyl acetate to polyvinyl alcohol is typically methanol which can remain in the resulting PVOH powder, even after drying. Upon dissolving the PVOH, the methanol is released into the atmosphere. Thus, it is desirable to reduce the methanol content remaining in the PVOH powder to less than 3 wt.%, or even less than 1 wt.%. Methods to remove volatile organic compounds include supplying a water-containing gas during the drying step, to replace the volatile organic compounds with water in the PVOH polymers. However, PVOH modified with monoesters, diesters, or anhydrides of ethylenically unsaturated dicarboxylic acids have a high affinity for water and the use of water-gas results in the dissolution of the surface of the powder particles and agglomeration of particles which makes processing the PVOH difficult. Alternatively, prolonged heating at high temperatures has been used to remove residual methanol. However, such high temperatures promote crosslinking between the PVOH hydroxyl moiety and the monoester, diester, and/or anhydride unit, resulting in insoluble components.

To reduce the amount of residual methanol in PVOH copolymers, the saponified copolymer is washed in a methanol/methyl acetate mixture having a methyl acetate content of about 45 vol.% or more, 60 vol.% or more, or 70 vol.% or more. For example, the PVOH gel obtained after the saponification step may be triturated with methanol/methyl acetate at a ratio of 15/85 (v/v).

Further, in order to reduce the amount of methanol, the particle size of the final PVOH resin can be reduced such that more than 95 wt.% pass through a 1.0 mm sieve, or more than 30% pass through a 500 micron sieve, or more than 45% pass through a 500 micron sieve. If the particle size of the final PVOH resin is too large, volatilization of the methanol becomes difficult.

For PVOH copolymer containing monoesters, diesters, or anhydrides of ethylenically unsaturated dicarboxylic acid, the amount of insoluble components can be reduced by controlling the ratio of lactone ring formation to copolymer modification by partial saponification. The ratio of lactone ring formation to copolymer modification can be described by the equation (Q):

$$0.05 \leq Y/X < 0.98 \qquad (Q)$$

wherein X is the copolymer modification and Y is the lactone ring formation. To reduce the amount of insoluble materials, Y/X is about 0.80 or less, about 0.60 or less, or about 0.40 or less.

When the PVOH resin comprises a PVOH copolymer including an anionic monomer, the level of incorporation of the one or more anionic monomer units in the PVOH copolymer is not particularly limited. In embodiments, the one or more anionic monomer units are present in the PVOH copolymer in an amount in a range of about 1 mol.% to 10 mol.%, or 1.5 mol.% to about 8 mol.%, or about 2 mol.% to about 6 mol.%, or about 3 mol.% to about 5 mol.%, or about 1 mol.% to about 4 mol.% (e.g., at least 1.0, 1.5, 2.0, 2.5, 3.0, 3.5, or 4.0 mol.% and/or up to about 3.0, 4.0, 4.5, 5.0, 6.0, 8.0, or 10 mol.% in various embodiments).

The water-soluble film contains at least 20 wt.%, 30 wt.%, 40 wt.%, 50 wt.%, 55 wt.%, 60 wt.%, 65 wt.%, 70 wt.%, 75 wt.%, 80 wt.%, 85 wt.% or 90 wt.% and/or up to about 60 wt.%, 70 wt.%, 80 wt.%, 90 wt.%, 95 wt.%, based on the total weight of the film, of the PVOH resin or resin blend. The PVOH resin or resin blend is present in the film in an amount of 20 wt.% to 95 wt.%, based on the total weight of the film. In embodiments, the PVOH resin or resin blend is present in the film in an amount of 60 wt.% to 95 wt.%, based on the total weight of the film. In embodiments, the PVOH resin or resin blend is present in the film in an amount of 85 wt.% to 95 wt.%, based on the total weight of the film.

In embodiments, the PVOH resin comprises a blend of PVOH polymers. In embodiments, the PVOH resin blend comprises an anionically modified PVOH polymer. In refinements of the foregoing embodiment, the PVOH resin blend further comprises a second PVOH polymer selected from a second anionically modified PVOH polymer or a PVOH homopolymer. In embodiments wherein the PVOH resin blend comprises two anionically modified PVOH polymers, the two anionically modified PVOH polymers can have the same anionic modification or different anionic modifications. For example, the two anionically modified PVOH polymers can each be a maleic modified polymer. Further, the two anionically modified PVOH polymers can be a maleic modified polymer and an acetate modified polymer, for example. In embodiments wherein the two anionically modified PVOH polymers have the same anionic modification, at least one property selected from viscosity, degree of hydrolysis, and/or degree of anionic modification can differ between the two anionically modified PVOH polymers.

### STARCH

The water-soluble film described herein comprises a hydroxypropyl-modified starch.

The starch can be provided in an amount of at least 2.5 phr, 5 phr, 6 phr, 7 phr, 10 phr, 12 phr, 15 phr, 20 phr, or 22phr and/or up to 5 phr, 10 phr, 12 phr, 16 phr, 20 phr, 22 phr, 24 phr, 26 phr, or 30 phr, based on the total amount of PVOH resin. For example, the starch can be provided in an amount in a range of 2.5 phr to 30 phr, or 5 phr to 30 phr, or 5 phr to 30 phr, or 5 phr to 15 phr, or 6 phr to 10 phr, or 12 phr to 16 phr, or 2 phr to 5 phr, or 5 phr to 12 phr, or 2.5 phr, 3 phr, 7 phr, 8phr, 9 phr, 13 phr, 14 phr, 16 phr, 20 phr, 22 phr, 24 phr, 25 phr, or 26 phr.

The starch can have an amylose content of at least 23%, 25%, 30%, 40%, 50%, 60%, 65%, or 75% and/or up to 60%, 70%, 80%, 85%, or 95%, for example, in a range of 65% to 95%, or 75% to 85%, or 25% to 70%, or 30% to 70%, or 40% to 60%, or 23% to 95%.

Sources of starch with an amylose content of between about 20% to about 80% include acorn, apple, arrowroot, barley, ester lily, elm tree, sapwood, iris tuber, maize, amylomaize, oat, pea, potato, sago and wheat. Sources of starch with an amylose content of between about 23% to about 95% include acorn, apple, ester lily, iris tuber, maize, amylomaize, oat, pea, sago and wheat.

Determination of amylose content can be accomplished by iodine complex formation as discussed in Amylose and Amylopectin Content of Starches Determined by their Iodine Complex Formation, F.L. Bates, D. French, and R.E. Rundle, J. Am. Chem. Soc., 1943, 65 (2), pp 142-148.

High amylose starches containing less than 45% amylopectin are more suitably measured with a modified potentiometric iodine method outlined in U.S. Patent No. 5,300,145 (incorporated herein by reference), Col. 8, line 14 - Col. 9, line 68. High amylose starches having an amylose amount above 55%, above 70% and above 89% are embodiments included herein.

The amylopectin present in starches suitable herein may include a high molecular weight fraction, an intermediate molecular weight fraction, and a low molecular weight fraction. The molecular weight fraction can be determined by gel-permeation chromatography discussed in the Journal of Cereal Science 27 (1998) 289-299, Shi, Y., Capitani, T., Trzasko, P., and Jeffoat, R. High amylose starches having an amylopectin content with all three weight fractions is an embodiment included herein. For example, a high amylose starch may include a high molecular weight fraction of amylopectin between 2-32% (such as 31.1%), an intermediate molecular weight fraction of linear and branched (mix of amylopectin and amylose) between 59-71% (such as 59.5%), and a low molecular weight fraction (amylose) between 9-27% (such as 9.4%).

The average particle size of the starch should not exceed 6 microns when in its solid state, for example, the starch can be provided in a range of 1 to 6 microns, 1 micron to 5.5 microns, 1.5 microns to 5 microns, 2 microns to 5 microns, 2 microns to 4 microns, 3 microns to 5 microns, 3 microns to 4 microns, for example, 1, 2, 3, 4, 5, or 6 microns. Without intending to be bound by theory, it is believed that as the average particle size of the starch increases beyond 6 microns, the physical properties of the resulting film, such as tensile strength and elongation, are adversely affected. It is common knowledge in the art that in the presence of water and heat starch will increase in size to some degree by swelling (e.g., from about 6 microns to about 8 microns), and then break down over time. Accordingly, as used herein, the "average particle size" of the starch refers to the dry particles of starch raw material. Methods for determining the average particle size of dry starch are well known in the art.

When starch in water is heated to temperatures below the starch gelatinization temperature, the starch will swell. When starch in water is heated to a temperature greater than or equal to the starch gelatinization temperature, the starch will break down and form a gel. The gelatinization temperature of a given starch depends on a variety of properties of the starch, including but not limited to the plant type the starch is sourced from, the degree of crosslinking in the starch, the degree of modification of the starch, and the degree of amylose content. In embodiments, the starch has a gelatinization temperature higher than the temperature at which the water-soluble film raw materials are mixed in water. In embodiments, the gelatinization temperature of the starch in water is greater than 75°C, greater than 80 °C, greater than 85°C, greater than 90°C, or greater than 95°C and up to 300°C, up to 250°C, 200°C, or 150°C, for example, in a range of 80°C to 200°C, or 85°C to 150°C. The presence of other chemicals in an aqueous solution can affect the gelatinization properties of the starch.

The starch can have a degree of modification of at least 2%, 4%, 5%, or 6% and/or up to 4%, 6%, 7%, or 8%, for example, greater than 2% or in a range of 2% to 8%, 4% to 8%, or 5% to 7%.

In embodiments, two or more starches can be used together to form a starch blend. Surprisingly, it was found that when a blend of starches was used, each starch in the blend independently influenced the properties of the final film in the same way each starch would if used as the sole starch. Accordingly, each starch of a blend independently can have a degree of modification, gelatinization temperature, particle size, and/or amylose content as described herein.

In embodiments, the starch comprises a hydroxypropylated starch and is present in the water-soluble film in an amount in a range of 5 phr to 30 phr, optionally 6 phr to 10 phr or 12 phr to 16 phr. Optionally, the hydroxypropylated starch has an amylose content in a range of 65% to 95% or 75% to 85%. Optionally, the hydroxypropylated starch has a degree of modification of 4% to 8% or 5% to 7%. In embodiments, a water-soluble film comprising a hydroxypropylated starch and having a thickness of 76 microns has a cold water solubility of less than 90 seconds as measured by MSTM-205.

Without intending to be bound by theory, one of ordinary skill in the art would expect that as the amount of starch present in the film increases, the mechanical properties of the film (e.g., tensile strength and tear strength) decreases. As demonstrated below, when an unmodified starch is present in a water-soluble film in a PVOH resin to starch ratio of about 80:20 (i.e., about 25 phr starch) or greater (e.g., 70:30), the tensile strength and tear strength of the film dramatically decrease relative to that of an equivalent water-soluble film including starch in a PVOH resin to starch ratio of less than 80:20, e.g., 97:3.

Advantageously it was surprisingly found that the mechanical properties of a film including a hydroxypropylated starch were maintained (i.e., did not significantly decrease) as the amount of hydroxypropylated starch included in the film increased over a range of 2.5 phr to 30 phr. In embodiments, the water-soluble film includes a hydroxypropylated starch present in an amount in a range of 5 phr to 30 phr, the film having a tensile strength of at least 40 MPa or at least 45 MPa to 60 MPa. Without intending to be bound by theory, it is believed that when the modified starch is a hydroxyalkyl modified starch wherein the alkyl comprises 3 or more carbon atoms, for example, 3 to 8 carbon atoms, the hydroxyalkyl chain disrupts the helical structure of the amylose, allowing for the amylose to more closely associate with the polyvinyl alcohol chains providing an increased mechanical strength to the resulting films, relative to a film including an unmodified starch.

Without intending to be bound by theory, it is believed that for a water-soluble film including a starch, as the amount of starch in the film increases, the G-G static coefficient of friction decreases. In embodiments, the water-soluble film includes a hydroxypropylated starch in a range of 5 phr to 30 phr, and the film has a G-G static coefficient of friction of 5 or less, 3 or less, 2 or less, or 1 or less. In embodiments, the water-soluble film includes a hydroxypropylated starch in a range of 5 phr to 30 phr, and the film is characterized by a reduced gloss to gloss static coefficient of friction compared to the equivalent film formulation that does not include a starch.

In embodiments, the starch is a modified starch having a degree of modification of greater than 2% and the modified starch is present in an amount in a range of 2.5 phr to 30 phr, optionally 6 phr to 16 phr, or 6 phr to 10 phr, or 12 phr to 16 phr. Optionally, the degree of modification is 4% to 8% or 5% to 7%. The modified starch has an amylose content in a range of 65% to 95% or 75% to 85%. The modified starch is a hydroxypropyl-modified starch. In embodiments, a water-soluble film comprising a modified starch having a degree of modification of greater than 2% and having a thickness of 76 microns has a cold water solubility of less than about 90 seconds as measured by MSTM-205.

Without intending to be bound by theory, it is believed that when the modified starch has a degree of modification of greater than 2% the mechanical properties of a film including the modified starch were maintained (i.e., did not significantly decrease) as the amount of starch included in the film increased over a range of 2.5 phr to 30 phr. In embodiments, the water-soluble film includes a modified starch having a degree of modification of greater than 2% present in an amount in a range of 2.5 phr to 30 phr, and the film has a tensile strength of at least 40 MPa or at least 45 MPa to 60 MPa. Without intending to be bound by theory, it is believed that when the modified starch has a degree of modification of greater than 2%, for example, 4% to 8%, the modifications disrupt the helical structure of the amylose, allowing for the amylose to more closely associate with the polyvinyl alcohol chains providing an increased mechanical strength to the resulting films, relative to a film including an unmodified starch.

In embodiments, the water-soluble film includes a modified starch having a degree of modification of greater than 2% in an amount in a range of 2.5 phr to 30 phr, the film has a G-G static coefficient of friction of 5 or less, 3 or less, 2 or less, or 1 or less. In embodiments, the water-soluble film includes a modified starch having a degree of modification of greater than 2% in an amount in a range of 2.5 phr to 30 phr, and the film is characterized by a reduced gloss to gloss static coefficient of friction compared to the equivalent film formulation that does not include a starch.

In embodiments, the water-soluble film includes a hydroxypropyl modified starch having an amylose content in a range of 23% to 95% by weight, based on the total weight of amylose and amylopectin in the hydroxypropyl-modified starch and the PVOH resin includes an unmodified polyvinyl alcohol, an anionic modified polyvinyl alcohol copolymer, or combinations thereof, with the proviso that the anionic modifier is not an acrylate. Optionally the hydroxypropylated starch is present in an amount in a range of 5 phr to 12 phr. Optionally, the hydroxypropylated starch has an amylose content in a range of 50% to 95%. Further optionally, the hydroxypropylated starch includes greater than 2% hydroxypropyl modifications, or 4% to 8% hydroxypropyl modifications. Without intending to be bound by theory, it is believed that the acrylate moiety negatively interacts with hydroxyalkyl modified starches resulting films having high G-G static coefficients of friction. In embodiments, the water-soluble film including a hydroxypropyl modified starch having an amylose content in a range of 23% to 95% and the PVOH resin includes an unmodified polyvinyl alcohol, an anionic modified polyvinyl alcohol copolymer, or combinations thereof, with the proviso that the anionic modifier is not an acrylate, has a gloss to gloss static coefficient of friction of 5 or less. In embodiments, the water-soluble film including a hydroxypropyl modified starch having an amylose content in a range of 23% to 95% and the PVOH resin includes an unmodified polyvinyl alcohol, an anionic modified polyvinyl alcohol copolymer, or combinations thereof, with the proviso that the anionic modifier is not an acrylate is characterized by a reduced gloss to gloss static coefficient of friction compared to the equivalent film formulation that does not include a starch.

## OTHER WATER-SOLUBLE POLYMERS

Other water-soluble polymers for use in addition to the PVOH resin or resin blend can include, but are not limited to a vinyl alcohol-vinyl acetate copolymer, sometimes referred to as a PVOH homopolymer, polyacrylates, water-soluble acrylate copolymers, polyvinyl pyrrolidone, polyethyleneimine, pullulan, water-soluble natural polymers including, but not limited to, guar gum, gum Acacia, xanthan gum, carrageenan, and starch (not previously discussed above), water-soluble polymer modified starches (not otherwise discussed above) including, but not limited to, ethylated starch, hydroxyethylated starch and hydroxypropylated starch, copolymers of the forgoing and combinations of any of the foregoing. Yet other water-soluble polymers can include polyalkylene oxides, polyacrylamides, polyacrylic acids and salts thereof, celluloses, cellulose ethers, cellulose esters, cellulose amides, polyvinyl acetates, polycarboxylic acids and salts thereof, polyaminoacids, polyamides, gelatines, methylcelluloses, carboxymethylcelluloses and salts thereof, dextrins, ethylcelluloses, hydroxyethyl celluloses, hydroxypropyl methylcelluloses, maltodextrins, polymethacrylates, and combinations of any of the foregoing. Such water-soluble polymers, whether PVOH or otherwise are commercially available from a variety of sources. In embodiments, the water-soluble film does not include polyacrylates, water-soluble acrylate copolymers, polyacrylic acids or salts thereof.

The water-soluble film can contain other auxiliary agents and processing agents, such as, but not limited to, plasticizers, plasticizer compatibilizers, surfactants, lubricants, release agents, fillers, extenders, cross-linking agents, antiblocking agents, antioxidants, detackifying agents, antifoams, nanoparticles such as layered silicate-type nanoclays (e.g., sodium montmorillonite), bleaching agents (e.g., sodium metabisulfite, sodium bisulfite or others), aversive agents such as bitterants (e.g., denatonium salts such as denatonium benzoate, denatonium saccharide, and denatonium chloride; sucrose octaacetate; quinine; flavonoids such as quercetin and naringen; and quassinoids such as quassin and brucine) and pungents (e.g., capsaicin, piperine, allyl isothiocyanate, and resinferatoxin), and other functional ingredients, in amounts suitable for their intended purposes. Embodiments including plasticizers are preferred. In embodiments, the water-soluble film includes a surfactant, an antioxidant, a bittering agent, a soil release polymer, an anti-redeposition aid, a chelant, a builder, a perfume, or combinations thereof. The amount of auxiliary agents can be up to about 50 wt. %, 20 wt %, 15 wt %, 10 wt %, 5 wt. %, 4 wt % and/or at least 0.01 wt. %, 0.1 wt %, 1 wt %, or 5 wt %, individually or collectively.

In methods, such as solvent casting, wherein the water-soluble film components are added to a solvent and are mixed or is held at a high temperature, the composition may undergo a browning. Such a browning is undesirable for most water-soluble film applications, as a clear film is sought. Therefore, the methods and compositions described herein can further include addition of a bisulfite or metabisulfite to the composition in an amount in a range of about 0.10 wt.% to about 0.75 wt.%, based on the weight of the resulting film, for example, in a range of about 0.4 wt.% to about 0.7 wt.%, to prevent browning of the heated composition.

## **PLASTICIZERS**

A plasticizer is a liquid, solid, or semi-solid that is added to a material (usually a resin or elastomer) making that material softer, more flexible (by decreasing the glass-transition temperature of the polymer), and easier to process. A polymer can alternatively be internally plasticized by chemically modifying the polymer or monomer. In addition or in the alternative, a polymer can be externally plasticized by the addition of a suitable plasticizing agent.

The plasticizer can include, but is not limited to, glycerol, diglycerin, sorbitol, ethylene glycol, diethylene glycol, triethylene glycol, dipropylene glycol, tetraethylene glycol, propylene glycol, polyethylene glycols up to 400 MW, neopentyl glycol, trimethylolpropane, polyether polyols, 2-methyl-1,3-propanediol (MPDiol®), ethanolamines, glycerol propylene oxide polymers (such as, for example, Voranol™ available from The Dow Chemical Company), and a mixture thereof. In embodiments, the plasticizer is selected from the group consisting of glycerol, propylene glycol, sorbitol, trimethylolpropane, polyethylene glycols up to 400 MW, glycerol propylene oxide polymers (such as, for example, Voranol™ available from The Dow Chemical Company), 2-methyl-1,3-propanediol, xylitol, and combinations of the foregoing. In embodiments, the plasticizer is selected from the group consisting of glycerol, sorbitol, 2-methyl-1,3-propanediol, and a combination thereof.

The total amount of the plasticizer can be in a range of about 10 wt. % to about 45 wt. %, or about 15 wt. % to about 35 wt. %, or about 20 wt. % to about 30 wt. %, or about 20 wt.% to about 45 wt.%, for example about 25 wt. %, based on total film weight. In embodiments, the amount of plasticizer in the water-soluble film is expressed in parts per 100 parts total water-soluble polymer (PHR) in the water-soluble film and is present in an amount of at least 1 PHR, at least 5 PHR, at least 10 PHR, at least 15 PHR, at least 20 PHR, at least 25 PHR, at least 30 PHR, or at least 35 PHR, for example. The total amount of plasticizer can be up to 40 PHR or 45 PHR or 50 PHR, for example. The total amount of plasticizer can be in a range of 1 PHR to 40 PHR, or 10 PHR to 40 PHR, or 30 PHR to 50 PHR, or 32.5 PHR to 42.5 PHR, or 35 PHR to 45 PHR, or 35 PHR to 40 PHR, for example. The specific amounts of plasticizers can be selected in a particular embodiment based on desired film flexibility and processability features of the water-soluble film. At low plasticizer levels, films may become brittle, difficult to process, or prone to breaking. At elevated plasticizer levels, films may be too soft, weak, or difficult to process for a desired use.

SURFACTANTS

Surfactants for use in water-soluble films are well known in the art. Optionally, surfactants are included to aid in the dispersion of the resin solution upon casting. Suitable surfactants can include the nonionic, cationic, anionic and zwitterionic classes. Suitable surfactants include, but are not limited to, polyoxyethylenated polyoxypropylene glycols, alcohol ethoxylates, alkylphenol ethoxylates, tertiary acetylenic glycols and alkanolamides (nonionics), polyoxyethylenated amines, quaternary ammonium salts and quaternized polyoxyethylenated amines (cationics), and amine oxides, N-alkylbetaines and sulfobetaines (zwitterionics). Other suitable surfactants include dioctyl sodium sulfosuccinate, lactylated fatty acid esters of glycerin and propylene glycol, lactylic esters of fatty acids, sodium alkyl sulfates, polysorbate 20, polysorbate 60, polysorbate 65, polysorbate 80, lecithin, acetylated fatty acid esters of glycerin and propylene glycol, and acetylated esters of fatty acids, and combinations thereof. In embodiments, the surfactant is selected from the group consisting of polyoxyethylenated polyoxypropylene glycols, alcohol ethoxylates, alkylphenol ethoxylates, tertiary acetylenic glycols and alkanolamides, polyoxyethylenated amines, quaternary ammonium salts and quaternized polyoxyethylenated amines, and amine oxides, N-alkylbetaines, sulfobetaines, and combinations thereof.

In various embodiments, the amount of surfactant in the water-soluble film is in a range of about 0.1 wt % to about 8.0 wt %, or about 1.0 wt % to about 7.0 wt %, or about 3 wt% to about 7 wt%, or about 5 wt% to about 7 wt%. In embodiments, the amount of surfactant in the water-soluble film is expressed in parts per 100 parts total water-soluble polymer (phr) in the water-soluble film and is present in a range of about 0.5 phr to about 12 phr, about 1.0 phr to about 11.0 phr, about 3.0 phr to about 10.5 phr, or about 1.0 phr to about 2.0 phr.

Suitable lubricants/release agents can include, but are not limited to, fatty acids and their salts, fatty alcohols, fatty esters, fatty amines, fatty amine acetates and fatty amides. Preferred lubricants/release agents are fatty acids, fatty acid salts, and fatty amine acetates. In one type of embodiment, the amount of lubricant/release agent in the water-soluble film is in a range of about 0.02 wt % to about 1.5 wt %, optionally about 0.1 wt % to about 1 wt %.

Suitable fillers/extenders/antiblocking agents/detackifying agents include, but are not limited to, crosslinked polyvinylpyrrolidone, crosslinked cellulose, microcrystalline cellulose, silica, metallic oxides, calcium carbonate, talc, mica, stearic acid and metal salts thereof, for example, magnesium stearate. Optionally an additional unmodified starch or modified starch can be included the water-soluble in addition to one of the specific starch components described above, for example, hydroxypropylated starch present in an amount in a range of 5 phr to 30 phr, or modified starch having a degree of modification of greater than 2% and is present in an amount in a range of 2.5 phr to 30 phr, or an unmodified starch having an amylose content in a range of 20% to 80%, or a hydroxypropyl modified starch having an amylose content in a range of 23% to 95% when the polyvinyl alcohol comprises an unmodified polyvinyl alcohol or an anionic modified polyvinyl alcohol copolymer with the proviso that the anionic modifier is not an acrylate. Preferred materials are starches, modified starches and silica. In one type of embodiment, the amount of filler/extender/antiblocking agent/detackifying agent in the water-soluble film can be in a range of about 1 wt.% to about 6 wt.%, or about 1 wt.% to about 4 wt.%, or about 2 wt.% to about 4 wt.%, or about 1 phr to about 6 phr, or about 1 phr to about 4 phr, or about 2 phr to about 4 phr, for example. In embodiments, when a starch or modified starch is included in the water-soluble film in addition to one of the specific starch components described above, the additional starch component will be provided in an amount of less than about 50 wt.%, based on the total weight of all starches included in the film. Without intending to be bound by theory, it is believed that any benefit provided to the water-soluble films of the disclosure from the inclusion of the starch component described above is not affected by including an additional starch component that provides a lesser benefit to the water-soluble film or no benefit to the water-soluble film.

The water-soluble film can further have a residual moisture content of at least 4 wt. %, for example in a range of about 4 to about 10 wt. %, as measured by Karl Fischer titration.

METHOD OF MAKING FILM

One contemplated class of embodiments is characterized by the water-soluble film of the water-soluble packets being formed by, for example, solvent casting. Processes for solvent casting of PVOH are well-known in the art. For example, in the film-forming process, the polyvinyl alcohol polymers and secondary additives are dissolved in a solvent, typically water, metered onto a surface, allowed to substantially dry (or force-dried) to form a cast film, and then the resulting cast film is removed from the casting surface. The process can be performed batchwise, and is more efficiently performed in a continuous process. For example, the water or solvent is heated to a temperature in a range of about 80 to 90°C, plasticizers, starches, and secondary additives are first added to the water or solvent, followed by the addition of surfactants. The mixture is stirred until a homogenous mixture is formed. The film forming resins are then added to mixture and mixed at about 80 to 90°C until homogenous.

In the formation of continuous films of polyvinyl alcohol, it is the conventional practice to meter a solution of the solution onto a moving casting surface, for example, a continuously moving metal drum or belt, causing the solvent to be substantially removed from the liquid, whereby a self-supporting cast film is formed, and then stripping the resulting cast film from the casting surface.

Optionally, the water-soluble film can be a free-standing film consisting of one layer or a plurality of like layers.

Residual moisture content

The water-soluble film can further have a residual moisture content of at least 4 wt. %, for example in a range of about 4 to about 10 wt. %, as measured by Karl Fischer titration.

POUCHES/PACKETS

The water-soluble film disclosed herein is useful for creating a sealed article in the form of a pouch defining an interior pouch volume to contain a composition therein. A "sealed article" encompasses sealed compartments having a vent hole, for example, in embodiments wherein the compartment encloses a solid that off-gasses. The pouch composition may take any form such as powders, gels, pastes, liquids, tablets or any combination thereof. The film is also useful for any other application in which improved wet handling and low cold water residues are desired.

The film described herein can also be used to make a packet with two or more compartments made of the same film or in combination with films of other polymeric materials. Additional films can, for example, be obtained by casting, blow-molding, extrusion or blown extrusion of the same or a different polymeric material, as known in the art. In one type of embodiment, the polymers, copolymers or derivatives thereof suitable for use as the additional film are selected from polyvinyl alcohols, polyvinyl pyrrolidone, polyalkylene oxides, polyacrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatin, natural gums such as xanthan, and carrageenans. For example, polymers can be selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and combinations thereof, or selected from polyvinyl alcohols, polyvinyl alcohol copolymers and hydroxypropyl methyl cellulose (HPMC), and combinations thereof. One contemplated class of embodiments is characterized by the level of polymer in the packet material, for example the PVOH copolymer described above, as described above, being at least 60%.

The pouches of the present disclosure can include at least one sealed compartment. Thus, the pouches may comprise a single compartment or multiple compartments. A water-soluble pouch can be formed from two layers of water-soluble polymer film sealed at an interface, or by a single film that is folded upon itself and sealed. One or both of the films include the PVOH film described above. The films define an interior pouch container volume which contains any desired composition for release into an aqueous environment. The composition is not particularly limited, for example including any of the variety of compositions described below. In embodiments comprising multiple compartments, each compartment may contain identical and/or different compositions. In turn, the compositions may take any suitable form including, but not limited to liquid, solid, pressed solids (tablets) and combinations thereof (e.g. a solid suspended in a liquid). In embodiments, the pouches comprises a first, second and third compartment, each of which respectively contains a different first, second, and third composition.

The compartments of multi-compartment pouches may be of the same or different size(s) and/or volume(s). The compartments of the present multi-compartment pouches can be separate or conjoined in any suitable manner. In embodiments, the second and/or third and/or subsequent compartments are superimposed on the first compartment. In embodiments, the second and/or third and/or subsequent compartments are orientated side-by-side or in concentric orientations. The compartments may be packed in a string, each compartment being individually separable by a perforation line. Hence each compartment may be individually torn-off from the remainder of the string by the end-user.

The geometry of the compartments may be the same or different. In embodiments the optionally third and subsequent compartments each have a different geometry and shape as compared to the first and second compartment. In these embodiments, the optionally third and subsequent compartments are arranged in a design on the first or second compartment. The design may be decorative, educative, or illustrative, for example to illustrate a concept or instruction, and/or used to indicate origin of the product.

The pouches and/or packets of the present disclosure may comprise one or more different films. For example, in single compartment embodiments, the packet may be made from one wall that is folded onto itself and sealed at the edges, or alternatively, two walls that are sealed together at the edges. In multiple compartment embodiments, the packet may be made from one or more films such that any given packet compartment may comprise walls made from a single film or multiple films having differing compositions.

Pouches and packets may be made using any suitable equipment and method. For example, single compartment pouches may be made using vertical form filling, horizontal form filling, or rotary drum filling techniques commonly known in the art. Such processes may be either continuous or intermittent. The film may be dampened, and/or heated to increase the malleability thereof. The method may also involve the use of a vacuum to draw the film into a suitable mold. The vacuum drawing the film into the mold can be applied for about 0.2 to about 5 seconds, or about 0.3 to about 3, or about 0.5 to about 1.5 seconds, once the film is on the horizontal portion of the surface. This vacuum can be such that it provides an under-pressure in a range of 10 mbar to 1000 mbar, or in a range of 100 mbar to 600 mbar, for example.

The molds, in which packets may be made, can have any shape, length, width and depth, depending on the required dimensions of the pouches. The molds may also vary in size and shape from one to another, if desirable. For example, the volume of the final pouches may be about 5 ml to about 300 ml, or about 10 ml to 150 ml, or about 20 ml to about 100 ml, and that the mold sizes are adjusted accordingly.

SHAPING, SEALING, AND THERMOFORMING

A thermoformable film is one that can be shaped through the application of heat and a force.

Thermoforming a film is the process of heating the film, shaping it (e.g. in a mold), and then allowing the film to cool, whereupon the film will hold its shape, e.g. the shape of the mold. The heat may be applied using any suitable means. For example, the film may be heated directly by passing it under a heating element or through hot air, prior to feeding it onto a surface or once on a surface. Alternatively, it may be heated indirectly, for example by heating the surface or applying a hot item onto the film. In embodiments, the film is heated using an infrared light. The film may be heated to a temperature in a range of about 50 to about 150 °C, about 50 to about 120 °C, about 60 to about 130 °C, about 70 to about 120 °C, or about 60 to about 90 °C. Thermoforming can be performed by any one or more of the following processes: the manual draping of a thermally softened film over a mold, or the pressure induced shaping of a softened film to a mold (e.g., vacuum forming), or the automatic high-speed indexing of a freshly extruded sheet having an accurately known temperature into a forming and trimming station, or the automatic placement, plug and/or pneumatic stretching and pressuring forming of a film.

Alternatively, the film can be wetted by any suitable means, for example directly by spraying a wetting agent (including water, a solution of the film composition, a plasticizer for the film composition, or any combination of the foregoing) onto the film, prior to feeding it onto the surface or once on the surface, or indirectly by wetting the surface or by applying a wet item onto the film.

Once a film has been heated and/or wetted, it may be drawn into an appropriate mold, preferably using a vacuum. The filling of the molded film can be accomplished by utilizing any suitable means. In embodiments, the most preferred method will depend on the product form and required speed of filling. In embodiments, the molded film is filled by in-line filling techniques. The filled, open packets are then closed forming the pouches, using a second film, by any suitable method. This may be accomplished while in horizontal position and in continuous, constant motion. The closing may be accomplished by continuously feeding a second film, preferably water-soluble film, over and onto the open packets and then preferably sealing the first and second film together, typically in the area between the molds and thus between the packets.

Any suitable method of sealing the packet and/or the individual compartments thereof may be utilized. Non-limiting examples of such means include heat sealing, solvent welding, solvent or wet sealing, and combinations thereof. Typically, only the area which is to form the seal is treated with heat or solvent. The heat or solvent can be applied by any method, typically on the closing material, and typically only on the areas which are to form the seal. If solvent or wet sealing or welding is used, it may be preferred that heat is also applied. Preferred wet or solvent sealing/welding methods include selectively applying solvent onto the area between the molds, or on the closing material, by for example, spraying or printing this onto these areas, and then applying pressure onto these areas, to form the seal. Sealing rolls and belts as described above (optionally also providing heat) can be used, for example.

The formed pouches may then be cut by a cutting device. Cutting can be accomplished using any known method. It may be preferred that the cutting is also done in continuous manner, and preferably with constant speed and preferably while in horizontal position. The cutting device can, for example, be a sharp item, or a hot item, or a laser, whereby in the latter cases, the hot item or laser 'burns' through the film/ sealing area.

The different compartments of a multi-compartment pouches may be made together in a side-by-side style or concentric style wherein the resulting, cojoined pouches may or may not be separated by cutting. Alternatively, the compartments can be made separately.

In embodiments, pouches may be made according to a process comprising the steps of: a) forming a first compartment (as described above); b) forming a recess within or all of the closed compartment formed in step (a), to generate a second molded compartment superposed above the first compartment; c) filling and closing the second compartments by means of a third film; d) sealing the first, second and third films; and e) cutting the films to produce a multi-compartment pouch. The recess formed in step (b) may be achieved by applying a vacuum to the compartment prepared in step (a).

In embodiments, second, and/or third compartment(s) can be made in a separate step and then combined with the first compartment as described in European Patent Application Number 08101442.5 or U.S. Patent Application Publication No. 2013/240388 A1 or WO 2009/152031.

In embodiments, pouches may be made according to a process comprising the steps of: a) forming a first compartment, optionally using heat and/or vacuum, using a first film on a first forming machine; b) filling the first compartment with a first composition; c) optionally filling the second compartment with a second composition; d) sealing the first and optional second compartment with a second film to the first film; and e) cutting the films to produce a multi-compartment pouch.

In embodiments, pouches may be made according to a process comprising the steps of: a) forming a first compartment, optionally using heat and/or vacuum, using a first film on a first forming machine; b) filling the first compartment with a first composition; c) on a second forming machine, deforming a second film, optionally using heat and vacuum, to make a second and optionally third molded compartment; d) filling the second and optionally third compartments; e) sealing the second and optionally third compartment using a third film; f) placing the sealed second and optionally third compartments onto the first compartment; g) sealing the first, second and optionally third compartments; and h) cutting the films to produce a multi-compartment pouch.

The first and second forming machines may be selected based on their suitability to perform the above process. In embodiments, the first forming machine is preferably a horizontal forming machine, and the second forming machine is preferably a rotary drum forming machine, preferably located above the first forming machine.

It should be understood that by the use of appropriate feed stations, it may be possible to manufacture multi-compartment pouches incorporating a number of different or distinctive compositions and/or different or distinctive liquid, gel or paste compositions.

In embodiments, the film and/or pouch is sprayed or dusted with a suitable material, such as an active agent, a lubricant, an aversive agent, or mixtures thereof. In embodiments, the film and/or pouch is printed upon, for example, with an ink and/or an active agent.

## VERTICAL FORM, FILL, AND SEALING

In embodiments, the water-soluble film of the disclosure can be formed into a sealed article. In embodiments, the sealed article is a vertical form, filled, and sealed article. The vertical form, fill, and seal (VFFS) process is a conventional automated process. VFFS includes an apparatus such as an assembly machine that wraps a single piece of the film around a vertically oriented feed tube. The machine heat seals or otherwise secures the opposing edges of the film together to create the side seal and form a hollow tube of film. Subsequently, the machine heat seals or otherwise creates the bottom seal, thereby defining a container portion with an open top where the top seal will later be formed. The machine introduces a specified amount of flowable product into the container portion through the open top end. Once the container includes the desired amount of product, the machine advances the film to another heat sealing device, for example, to create the top seal. Finally, the machine advances the film to a cutter that cuts the film immediately above the top seal to provide a filled package.

During operation, the assembly machine advances the film from a roll to form the package. Accordingly, the film must be able to readily advance through the machine and not adhere to the machine assembly or be so brittle as to break during processing.

Water-soluble films may be converted by VFFS on a Rovema VFFS machine or equivalent. Pouches/packets are prepared in the standard laboratory atmosphere of 78°F (about 25.5°C) and 24 % relative humidity. Both unfilled bags and bags filled with salt are produced. The salt-containing bags are filled to 1.2 lbs (about 550 g) at max fill level. The horizontal seal temperature is 225°F (about 107°C), and the vertical seal temperature is varied as needed to produce a good seal, for example, between about 255°F (about 124°C) and 280°F (about 138°C).

POUCH CONTENTS

The present articles (e.g., in the form of pouches or packets) may contain various compositions, for example household care compositions (detergent compositions) or non-household care compositions. A multi-compartment pouch may contain the same or different compositions in each separate compartment. The composition is proximal to the water-soluble film. The composition may be less than about 10 cm, or less than about 5 cm, or less than about 1cm from the film. Typically the composition is adjacent to the film or in contact with the film. The film may be in the form of a pouch or a compartment, containing the composition therein.

An inner film of the pouch may be utilized as a partition to keep compositions containing incompatible ingredients physically separated from each other. It is believed that such partitioning may expand the useful life and/or decrease physical instability of such ingredients. Additionally or alternatively, such partitioning may provide aesthetic benefits as described in U.S. Patent Number 8,835,372.

In embodiments, the composition may be selected from the group of detergent compositions as liquid light duty and liquid heavy duty liquid detergent compositions, powdered detergent compositions, dish detergent for hand washing and/or machine washing; hard surface cleaning compositions, fabric enhancers, detergent gels commonly used for laundry, and bleach and laundry additives, shaving creams, skin care, hair care compositions (shampoos and conditioners), and body washes. Such detergent compositions may comprise a surfactant, a bleach, an enzyme, a perfume, a dye or colorant, a solvent and combinations thereof.

In embodiments, the composition may be selected from the group of non-household care compositions including agricultural compositions, automotive compositions, aviation compositions, food and nutritive compositions, industrial compositions, livestock compositions, marine compositions, medical compositions, mercantile compositions, military and quasi-military compositions, office compositions, and recreational and park compositions, pet compositions, water-treatment compositions, including cleaning and detergent compositions applicable to any such use.

In embodiments, the composition contained in the interior pouch volume is a detergent composition. Optionally, the detergent composition is selected from the group consisting of a laundry detergent, a dishwashing detergent, a hard surface cleaning composition, a fabric enhancer composition, a shampoo, a body wash, and combinations thereof.

Non-limiting examples of useful non-household care compositions agricultural compositions, automotive compositions, aviation compositions, food and nutritive compositions, industrial compositions, livestock compositions, marine compositions, medical compositions, mercantile compositions, military and quasi-military compositions, office compositions, and recreational and park compositions, pet compositions, water-treatment compositions, including cleaning and detergent compositions applicable to any such use. Compositions of use in the present pouches may take the form of a liquid, solid or a powder. Liquid compositions may comprise a solid. Solids may include powder or agglomerates, such as micro-capsules, beads, noodles or one or more pearlized balls or mixtures thereof. Such a solid element may provide a technical benefit, through the wash or as a pre-treat, delayed or sequential release component; additionally or alternatively, it may provide an aesthetic effect (e.g., perfume microcapsules).

The compositions encapsulated by the films described herein, when liquid compositions, can have any suitable viscosity depending on factors such as formulated ingredients and purpose of the composition. In one embodiment, the composition has a high shear viscosity value, at a shear rate of $20s^{-1}$ and a temperature of 20°C, of 100 to 3,000 cP, alternatively 300 to 2,000 cP, alternatively 500 to 1,000 cP, and a low shear viscosity value, at a shear rate of $1 s^{-1}$ and a temperature of 20°C, of 500 to 100,000 cP, alternatively 1000 to 10,000 cP, alternatively 1,300 to 5,000 cP. Methods to measure viscosity are known in the art. According to the present invention viscosity measurements are carried out using a rotational rheometer e.g. TA instruments AR550. The instrument includes a 40mm 2° or 1° cone fixture with a gap of around 50-60$\mu$m for isotropic liquids, or a 40mm flat steel plate with a gap of 1000 $\mu$m for particles containing liquids. The measurement is carried out using a flow procedure that contains a conditioning step, a peak hold and a continuous ramp step. The conditioning step involves the setting of the measurement temperature at 20°C, a pre-shear of 10 seconds at a shear rate of $10s^{-1}$, and an equilibration of 60 seconds at the selected temperature. The peak hold involves applying a shear rate of $0.05s^{-1}$ at 20°C for 3min with sampling every 10s. The continuous ramp step is performed at a shear rate from 0.1 to $1200s^{-1}$ for 3min at 20°C to obtain the full flow profile.

As described above, the composition may be a non-household care composition. For example, a non-household care composition can be selected from agricultural compositions, automotive compositions, aviation compositions, food and nutritive compositions, industrial compositions, livestock compositions, marine compositions, medical compositions, mercantile compositions, military and quasi-military compositions, office compositions, and recreational and park compositions, pet compositions, water-treatment compositions, including cleaning and detergent compositions applicable to any such use.

In one type of embodiment, the composition can include an agrochemical, e.g. one or more insecticides, fungicides, herbicides, pesticides, miticides, repellants, attractants, defoliaments, plant growth regulators, fertilizers, bactericides, micronutrients, and trace elements. Suitable agrochemicals and secondary agents are described in U.S. Patent Nos. 6,204,223 and 4,681,228 and EP 0989803 A1. For example, suitable herbicides include paraquat salts (for example paraquat dichloride or paraquat bis(methylsulphate), diquat salts (for example diquat dibromide or diquat alginate), and glyphosate or a salt or ester thereof (such as glyphosate isopropylammonium, glyphosate sesquisodium or glyphosate trimesium, also known as sulfosate). Incompatible pairs of crop protection chemicals can be used in separate chambers, for example as described in U.S. Patent No. 5,558,228. Incompatible pairs of crop protection chemicals that can be used include, for example, bensulfuron methyl and molinate; 2,4-D and thifensulfuron methyl; 2,4-D and methyl 2-[[[[N-4-methoxy-6-methyl-1,3,5-triazine-2-yl)-N-methylamino]carbonyl]amino]-sulfonyl]benzoate; 2,4-D and metsulfuron methyl; maneb or mancozeb and benomyl; glyphosate and metsulfuron methyl; tralomethrin and any organophosphate such as monocrotophos or dimethoate; bromoxynil and N-[[4,6-dimethoxypyrimidine-2-yl) -amino]carbonyl]-3-(ethylsulfonyl)-2-pyridine - sulfonamide; bromoxynil and methyl 2-[[[[(4-methyl-6-methoxy)-1,3,5-triazin-2-yl)amino]carbonyl]amino]sulfonyl]-benzoate; bromoxynil and methyl 2-[[[[N-(4-methoxy-6-methyl-1,3,5-triazin-2-yl)-N-methylamino]carbonyl]amino]-sulfonyl]benzoate. In another, related, type of embodiment, the composition can include one or more seeds, optionally together with soil, and further optionally together with one or more additional components selected from mulch, sand, peat moss, water jelly crystals, and fertilizers, e.g. including types of embodiments described in U.S. Patent No. 8,333,033.

In another type of embodiment, the composition is a water-treatment agent. Such agents include aggressive oxidizing chemicals, e.g. as described in U.S. Patent Application Publication No. 2014/0110301 and U.S. Patent No. 8,728,593. For example, sanitizing agents can include hypochlorite salts such as sodium hypochlorite, calcium hypochlorite, and lithium hypochlorite; chlorinated isocyanurates such as dichloroisocyanuric acid (also referred to as "dichlor" or dichloro-s-triazinetrione, 1 ,3-dichloro- 1 ,3,5-triazinane-2,4,6-trione) and trichloroisocyanuric acid (also referred to as "trichlor" or 1,3,5-trichloro-1,3,5-triazinane-2,4,6-trione). Salts and hydrates of the sanitizing compounds are also contemplated. For example, dichloroisocyanuric acid may be provided as sodium dichloroisocyanurate, sodium dichloroisocyanurate acid dihydrate, among others. Bromine containing sanitizing agents may also be suitable for use in unit dose packaging applications, such as 1,3-dibromo-5,5-dimethylhydantoin (DBDMH), 2,2- dibromo-3-nitrilopropionamide (DBNPA), dibromocyano acetic acid amide, 1-bromo- 3-chloro-5,5-dimethylhydantoin; and 2-bromo-2-nitro- 1,3 -propanediol, among others. The oxidizing agent can be one described in U.S. Patent No. 7,476,325, e.g. potassium hydrogen peroxymonosulfate. The composition can be a pH-adjusting chemical, e.g. as described in U.S. Patent Application Publication No. 2008/0185347, and can include, for example, an acidic component and an alkaline component such that the composition is effervescent when contacted with water, and adjusts the water pH. Suitable ingredients include sodium bicarbonate, sodium bisulfate, potassium hydroxide, sulfamic acid, organic carboxylic acids, sulfonic acids, and potassium dihydrogen phosphate. A buffer blend can include boric acid, sodium carbonate, glycolic acid, and oxone monopersulfate, for example.

A water-treatment agent can be or can include a flocculant, e.g. as described in U.S. Patent Application Publication No. 2014/0124454. The flocculant can include a polymer flocculant, e.g. polyacrylamide, a polyacrylamide copolymer such as an acrylamide copolymers of diallydimethylammonium chloride (DADMAC), dimethylaminoethylacrylate (DMAEA), dimethylaminoethylmethacrylate (DMAEM), 3-methylamidepropyltrimethylammonium chloride (MAPTAC) or acrylic acid; a cationic polyacrylamide; an anionic polyacrylamide; a neutral polyacrylamide; a polyamine; polyvinylamine; polyethylene imine; polydimethyldiallylammonium chloride; poly oxyethylene; polyvinyl alcohol; polyvinyl pyrrolidone; polyacrylic acid; polyphosphoric acid; polystyrene sulfonic acid; or any combination thereof. A flocculant can be selected from chitosan acetate, chitosan lactate, chitosan adipate, chitosan glutamate, chitosan succinate, chitosan malate, chitosan citrate, chitosan fumarate, chitosan hydrochloride, and combinations thereof. The water-treating composition can include a phosphate removing substance, e.g. one or more selected from a zirconium compound, a rare earth lanthanide salt, an aluminum compound, an iron compound, or any combination thereof.

The composition can be a limescale removing composition, e.g. citric or maleic acid or a sulphate salt thereof, or any mixture thereof, e.g. as described in U.S. Patent Application No. 2006/0172910.

Various other types of compositions are contemplated for use in the packets described herein, including particulates, for example down feathers, e.g. as described in US RE29059 E; super absorbent polymers, e.g. as described in U.S. Patent Application Publication Nos. 2004/0144682 and 2006/0173430; pigments and tinters, e.g. as described in U.S. Patent No. 3,580,390 and U.S. Patent Application Publication No. 2011/0054111; brazing flux (e.g. alkali metal fluoroaluminates, alkali metal fluorosilicates and alkali metal fluorozincates), e.g. as described in U.S. Patent No. 8,163,104; food items (e.g., coffee powder or dried soup) as described in U.S. Patent Application Publication No. 2007/0003719; and wound dressings, e.g. as described in U.S. Patent No. 4,466,431.

**Dissolution and Disintegration Test (MSTM 205)**

A film can be characterized by or tested for Dissolution Time and Disintegration Time according to the MonoSol Test Method 205 (MSTM 205), a method known in the art. See, for example, U.S. Patent No. 7,022,656.

Apparatus and Materials:

1. 600 mL Beaker
2. Magnetic Stirrer (Labline Model No. 1250 or equivalent)
3. Magnetic Stirring Rod (5 cm)
4. Thermometer (0 to 100 °C ± 1 °C)
5. Template, Stainless Steel (3.8 cm x 3.2 cm)
6. Timer (0 - 300 seconds, accurate to the nearest second)
7. Polaroid 35 mm slide Mount (or equivalent)
8. MonoSol 35 mm Slide Mount Holder (or equivalent)
9. Distilled water

For each film to be tested, three test specimens are cut from a film sample using stainless steel template (i.e., 3.8 cm x 3.2 cm specimen). If cut from a film web, specimens should be cut from areas of web evenly spaced along the traverse direction of the web. Each test specimen is then analyzed using the following procedure.

1. Lock each specimen in a separate 35 mm slide mount.
2. Fill beaker with 500 mL of distilled water. Measure water temperature with thermometer and, if necessary, heat or cool water to maintain temperature at 20 °C (about 68 °F).
3. Mark height of column of water. Place magnetic stirrer on base of holder. Place beaker on magnetic stirrer, add magnetic stirring rod to beaker, turn on stirrer, and adjust stir speed until a vortex develops which is approximately one-fifth the height of the water column. Mark depth of vortex.
4. Secure the 35 mm slide mount in the alligator clamp of the 35 mm slide mount holder such that the long end of the slide mount is parallel to the water surface. The depth adjuster of the holder should be set so that when dropped, the end of the clamp will be 0.6 cm below the surface of the water. One of the short sides of the slide mount should be next to the side of the beaker with the other positioned directly over the center of the stirring rod such that the film surface is perpendicular to the flow of the water.
5. In one motion, drop the secured slide and clamp into the water and start the timer. Disintegration occurs when the film breaks apart. When all visible film is released from the slide mount, raise the slide out of the water while continuing to monitor the solution for undissolved film fragments. Dissolution occurs when all film fragments are no longer visible and the solution becomes clear.

The results should include the following: complete sample identification; individual and average disintegration and dissolution times; and water temperature at which the samples were tested.

Film disintegration times (I) and film dissolution times (S) can be corrected to a standard or reference film thickness using the exponential algorithms shown below in Equation 1 and Equation 2, respectively.

$$I_{corrected} = I_{measured} / (\text{reference thickness/measured thickness})^{1.83} \qquad [1]$$

$$S_{corrected} = S_{measured} / (\text{reference thickness/measured thickness})^{1.93} \qquad [2]$$

**Tensile Strength Test**

A film characterized by or to be tested for tensile strength according to the Tensile Strength Test is analyzed as follows. The procedure includes the determination of Tensile Strength according to ASTM D 882 ("Standard Test Method for Tensile Properties of Thin Plastic Sheeting") or equivalent. An INSTRON® tensile testing apparatus (Model 5544 Tensile Tester or equivalent) is used for the collection of film data. A minimum of three test specimens, each cut with reliable cutting tools to ensure dimensional stability and reproducibility, are tested in the machine direction (MD) (where applicable) for each measurement. Tests are conducted in the standard laboratory atmosphere of 23 ± 2.0°C and 35 ± 5 % relative humidity. For tensile strength determination, 1"-wide (2.54 cm) samples of a single film sheet having a thickness of 3.0 ± 0.15 mil (or 76.2 ± 3.8 μm) are prepared. The sample is then transferred to the INSTRON® tensile testing machine to proceed with testing while minimizing exposure in the 35% relative humidity environment. The tensile testing machine is prepared according to manufacturer instructions, equipped with a 500 N load cell, and calibrated. The correct grips and faces are fitted (INSTRON® grips having model number 2702-032 faces, which are rubber coated and 25 mm wide, or equivalent). The samples are mounted into the tensile testing machine and analyzed to determine the Tensile Strength (i.e., stress required to break film).

Suitable behavior of films according to the disclosure is marked by Tensile Strength values (in the machine direction (MD)) of at least 40 MPa as measured by the Tensile Strength Test. In various embodiments, the film has a Tensile Strength value of at least 40 MPa, and/or up to 60 MPa (e.g., 40, 45, 60, 55 or 60 MPa).

**Tear Strength Test**

This method covers the determination of the average force in grams per mil of specimen thickness required to propagate tearing through a specified length of polyvinyl alcohol (PVOH) film. The force in grams required to propagate tearing across a film is measured using a precisely calibrated pendulum device. Acting by gravity, the pendulum swings through an arc, tearing the specimen from a pre-cut slit. The specimen is held stationary on one side and on the other is fixed to the pendulum. The loss of energy of the pendulum swing is indicated by a pointer on a scale. The scale indication is a function of the force required to tear the specimen. This method is of value in ranking relative tearing resistance of PVOH films. The water-soluble films were evaluated on an Elmendorf Tearing Tester Model # 40043, in accordance with MSTM 107RD Standard Test Method for Propagation Tear Resistance of Polyvinyl Alcohol Film.

The water-soluble films were conditioned at a temperature of about 23 °C +/- 3 °C (73 °F +/- 5 °F) and relative humidity of about 35% ± 5% for not less than 8 hours prior to the test. The tests were conducted in the standard laboratory atmosphere of a temperature of 23 °C +/- 3 °C (73 °F +/- 5 °F) and a relative humidity of 35% ± 5%. The average tearing force in grams-force per MIL was calculated as follows:

$$\text{Tearing force, g/mil} = \frac{\text{(Augmenting Weight g/100) x scale reading}}{\text{Film thickness in MIL}}$$

Suitable behavior of films according to the disclosure is marked by Tear Strength values (in the machine direction (MD)) of at least about 800 g/mil as measured by the Tear Strength Test. In various embodiments, the film has a Tear Strength value (MD) of at least 500 g/mil, and/or up to about 2000 g/mil (e.g., about 500, about 800, about 1000, about 1200 or about 1500 g/mil).

**Coefficient Of Friction Test**

The Coefficient of Friction method tests the friction of two pieces of material that are rubbed against each other; the force required to move one piece against the other is measured. The force to start the sled (static friction) and the force to keep the sled moving (dynamic friction) are both measured by the load cell using ASTM D1894 "Friction Testing of Plastic Film and Sheeting."

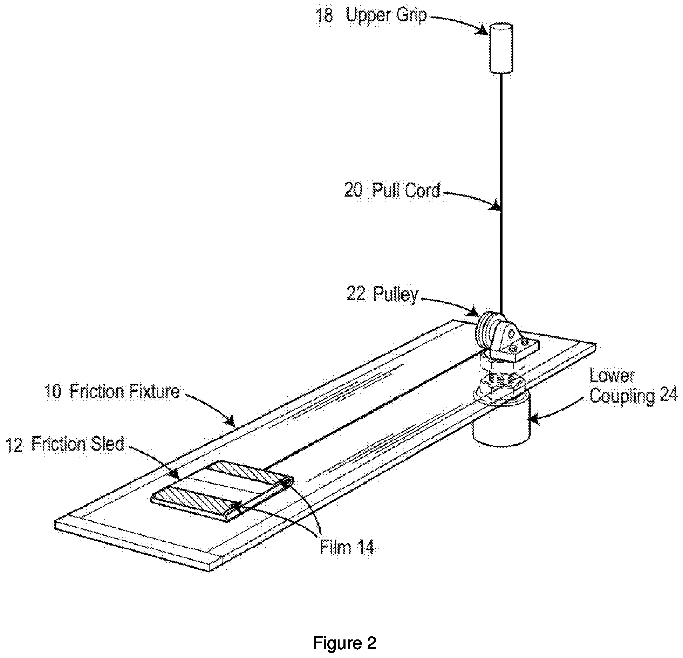

The method uses an Instron® Coefficient of Friction Testing Fixture Model 2810-005, or equivalent, a representative diagram of which is shown in Figure 2, and an Instron® Testing Machine Model # 5543, or equivalent.

The testing apparatus includes a friction fixture 10 upon which rests a friction sled 12 having secured thereon a film sample 14. The sled 12 is coupled to the upper grip 18 via a pull cord 20 which engages with pulley 22 secured to the friction fixture 10. The lower coupling 24 secures the testing fixture to the Instron® testing machine (not shown).

According the Instron® method Blue Hill program: "The system: searches the data from the start value to the end value on the specified channel for the maximum value; determines the first data point that rises and falls by the percentage of the maximum value and assigns this point as the first peak; uses the following equation to determine the coefficient of static friction: static friction = first peak/sled weight; uses the following equation to calculate the average load of the area from the first peak to the end value: average load = energy/change in extension; and uses the following equation to determine the coefficient of dynamic friction: dynamic friction = average load/sled weight."

The test specimen shall consist of samples having dimensions (5 inch by 5 inch square (12.7 cm by 12.7 cm square) for the sled and 5 inch by 8 inch rectangle (12.7 cm by 20.3 cm) for the surface, to form a testing area. While it is believed that the film thickness will not affect the Static COF, the film can have a thickness of 3.0 ± 0.10 mil (or 76.2 ± 2.5 μm). The samples can be cut using a razor blade and templates of the appropriate dimensions, for example. When applicable, the sample should be cut with the long dimension parallel to the machine direction of the cast film. Again when applicable, the 5 inch x 5 inch sample direction should be noted and oriented in the test so that the direction the sled is being pulled is parallel to the machine direction of the film sample.

The test specimen shall be conditioned at 75 ° F ± 5° F and relative humidity 35% ± 5% for not less than 8 hours prior to the test, and the test is conducted at the same temperature and relative humidity conditions.

Installation Procedure of COF apparatus

    1. Remove the clevis pin from the lower jaw on the Instron® Coefficient of Friction Testing Fixture Model 2810-005, and remove.

2. Remove the clevis pin from the upper jaw, and remove.

3. Place the friction fixture lower coupling onto the base adapter of the Instron® Testing Machine Model # 5543.

4. Fit it with the clevis pin.

5. Slip the loop of one end of the pull cord onto the upper clevis pin, and replace the locking clip.

6. Calibrate Testing Machine Model # 5543

7. Slip the loop on the other end of the pull cord onto the friction sled hook.

8. Make sure the pulley is able to spin freely

9. Move the sled till the pull cord has no slack and is oriented in the groove around the pulley.

10. Position the moving crosshead (upper heard) of the Instron® Coefficient of Friction Testing Fixture Model 2810-005 so that there is sufficient travel space to draw the friction sled along the full 50 mm of the test without running the sled into the pulley.

11. Keep the cord taught while the crosshead is moving.

12. Using the JOG control on the Instron #5543 control panel, set the extension limit so that the far end of the friction sled does not exceed the back plane (the plane perpendicular to the axis of motion, and furthest from the pulley) of the friction fixture. Press the GL button to set the travel limit. This prevents the friction sled from colliding with the pulley during the test, and insures that the coefficient of friction of the sample of interest is properly measured.

13. The test fixture is now ready for testing.

## PLACEMENT OF SPECIMEN PROCEDURE

1. Place the surface sample on the aluminum friction fixture in the appropriate orientation.

2. Pull the surface sample tight over the edges of the aluminum surface and tape the sample on the bottom side of the friction fixture.

3. It is important to tape along the end of the friction fixture furthest from the coupling to avoid binding of the sled on the surface.

4. Make sure that the material is taught but not stretched.

5. Wrap the friction sled with the 5X5 inch sample so that the machine direction of the film is parallel to the direction the sled will be pulled.

6. Tape the leading edge overlap on the top of the sled making sure there is no excess material which will bind up on the surface sample.

7. Tape the other edges of the sample on the friction sled to ensure the sample is taught on the contact surface being measured.

8. Be sure that no tape will get between the surface of interest on the sled and on the friction fixture.

9. The samples on the friction surface and on the friction sled should be taught with no wrinkles or bulges; these will cause errors in measuring the COF.

10. Inspect the sled to be sure there are no foreign materials touching the surfaces being tested.

11. Attach the sled to the pull cord and place the sled very lightly and gently on the friction table in order to prevent any unnatural bond from developing between the two specimens, begin test promptly.

12. Be sure that at full extension the sled sits completely over the sample placed on the friction fixture and does not contact tape or hang over the edge of the friction fixture.

## PERFORMING THE COF TEST

1. Test not less than three specimens per requested orientation (example air side - air side or band side - band side).

2. For a combination of air side to band side testing, the air side orientation of the film should be the film sample placed on the aluminum test surface, and the band side for testing should comprise the material wrapped around the sled.

3. Be sure to wear powder-free, moisture barrier gloves while handling the film specimens; powder or moisture may compromise the accuracy of the test.

4. Cut a sample as described above, e.g. using a template.

5. Place the friction sled wrapped in the first specimen at the end of the friction fixture furthest from the pulley.

6. Make sure the pull cord is pulled taught.

7. Open the Coefficient of Friction test titled "COF.im ptf" from the testing screen.

8. Click the start button on the screen to begin the test.

9. Upon completion of the specimen test run, click ok and return the friction sled to the starting position and change the film specimen on the friction sled and the fixture. Repeat the test.

The film can be characterized by a static COF in a range of 4.0 or less, or 2.0 or less, or 1.5 or less, or 1.25 or less, or 1.0 or less, for example 1.0, 0.9, 0.8, 0.7, 0.6, or even less. In another aspect, the static COF can be less than 4.0, or less than about 2.5, or less than 2, or less than 1.

In one or more aspects, the film can be characterized by having a G-G Static COF less than 5 and a tensile strength in a range of 40 to 60 MPa.

The water-soluble films in accordance with the disclosure can be better understood in light of the following examples, which are merely intended to illustrate the water-soluble films and are not meant to limit the scope thereof in any way.

## Examples

## Example 1

Water-soluble films including a water-soluble polyvinyl alcohol, a plasticizer and a modified starch were prepared. The film formulations included a polyvinyl alcohol copolymer having about a 4% maleate modification. Three film formulations further included about 26 phr plasticizers, minor amounts of processing aids e.g., surfactants, antioxidants, etc. totaling about 1 phr, and a hydroxypropylated starch having an 80% amylose content and about 6% hydroxypropyl modification present at a level of about 2.66 phr **(1a),** about 7.98 phr **(1b),** or 23.94 phr **(1c).** These films included about 77 wt.% **(1a),** about 74 wt.% **(1b),** and about 66 wt.% **(1c)** PVOH resin, based on the total weight of the film. Three additional film formulations included, in addition to the PVOH resin, about 26 phr plasticizers, minor amounts of processing aids e.g., surfactants, antioxidants, etc. totaling about 1 phr, and a hydroxyethylated starch having an amylose content of about 20% and about 2% hydroxypropyl modification present at a level of about 2.66 phr **(1d),** about 7.98 phr **(1e),** or 23.94 phr **(1f).** These films included about 77 wt.% **(1d),** about 74 wt.% **(1e),** and about 66 wt.% **(1f)** PVOH resin, based on the total weight of the film. Films have a thickness of about 75 microns were prepared. The films were tested for cold water solubility at 10°C according to MSTM-205, for tensile strength according to the Tensile Strength Test, and for coefficient of friction according to the Coefficient of Friction Test. Films that dissolved in 100 seconds or less are indicated as (+) in Table 1 and films that dissolved in 300 seconds or less but more than 100 seconds are indicated as (*) in Table 1, below. Films that had a tensile strength of about 45 MPa or greater are indicated as (+) in Table 1, films that had a tensile strength less than about 45 MPa but at least about 40 MPa or greater are indicated as (*) in Table 1, and films having a tensile strength of less than 40 MPa are indicated as (-) in Table 1, below. Films having a gloss-to-gloss static coefficient of friction of about 1 or less are indicated as (+) in Table 1, films having a gloss-to-gloss coefficient of friction of about 5 or less, but greater than about 1 are indicated as (*) in Table 1, and films having a gloss-to-gloss coefficient of friction of greater than about 5 are indicated as (-) in Table 1, below.

| Table 1 Sample (phr starch) | Solubility | Tensile Strength (MPa) | Coefficient of Friction |
|---|---|---|---|
| 1a (2.66) | + | + (49.84) | * |
| 1b (7.98) | + | + (55.78) | + |
| 1c (23.94) | + | + (48.48) | + |
| 1d (2.66) | + | + (55.44) | - |
| 1e (7.98) | + | + (52.22) | - |
| 1f (23.94) | + | +(44.87) | + |

Table 1 shows that films of the disclosure comprising a mixture of a water-soluble polyvinyl alcohol, plasticizer, and a hydroxypropylated starch present in an amount of about 5 phr to about 30 phr demonstrate good cold water solubility, good tensile strength, and excellent coefficient of frictions. Table 1 further shows that films of the disclosure comprising a mixture of a water-soluble polyvinyl alcohol, plasticizer, and a hydroxypropylated starch present in an amount of less than about 5 phr demonstrate good cold water solubility, good tensile strength, and acceptable coefficient of frictions. Table 1 further shows that the tensile strength of a water-soluble film comprising a hydroxypropylated starch is maintained or improved upon increasing the starch loading from about 2.5 phr to about 24 phr, whereas the tensile strength of a water-soluble film comprising a hydroxyethylated starch decreases with increasing starch loading.

Table 1 further shows that films of the disclosure comprising a mixture of a water-soluble polyvinyl alcohol, a plasticizer, and a modified starch having a degree of modification of greater than about 2% and present in an amount in a range of about 2.5 phr to about 30 phr demonstrate good cold water solubility, good tensile strength, and acceptable coefficient of frictions, and when provided in a range of about 5 to about 30 phr demonstrate a combination of good cold water solubility, good tensile strength, and excellent coefficient of frictions. Table 1 further shows that water-soluble films having a modified starch having a degree of modification of about 2% and present at low loading levels, e.g., about 12 phr or less, demonstrate increased coefficients of friction relative to films having modified starch having a degree of modification of greater than 2% at similar loading levels. Table 1 further shows that the tensile strength of a water-soluble film comprising a modified starch having a degree of modification of greater than about 2% is maintained or improved upon increasing the starch loading from about 2.5 phr to about 24 phr, whereas the tensile strength of a water-soluble film comprising a modified starch having a degree of modification of less than about 2% decreases with increasing starch loading.

Thus Example 1 demonstrates water-soluble films of the disclosure comprising a mixture of a water-soluble polyvinyl alcohol, plasticizer, and a hydroxypropylated starch present in an amount of about 5 phr to about 30 phr and water-soluble films of the disclosure comprising a mixture of a water-soluble polyvinyl alcohol, a plasticizer, and a modified starch having a degree of modification of greater than about 2% and present in an amount in a range of about 5 phr to about 30 phr that have good cold water solubility, good convertibility, and good film-to-film anti stick properties as demonstrated by a low G-G coefficient of friction.

## Example 2

Water-soluble films including a water-soluble polyvinyl alcohol, a plasticizer and an unmodified starch were prepared. The film formulations included a polyvinyl alcohol copolymer having about a 4% maleate modification. The film formulations further included about 26 phr plasticizers, minor amounts of processing aids e.g., surfactants, antioxidants, etc. totaling about 1 phr, and an unmodified starch present at a level of about 2.66 phr **(2a, 2d, 2g, 2j)**, about 7.98 phr **(2b, 2e, 2h, 2k)**, or 23.94 phr **(2c, 2f, 2i, 2l)**. Three films included an unmodified starch having an amylose content of 1% **(2a, 2b, 2c)**, three films included an unmodified starch having an amylose content of 25% **(2d, 2e, 2f)**, three films included an unmodified starch having an amylose content of 50% **(2g, 2h, 2i)**, and three films included an unmodified starch having an amylose content of 80% **(2j, 2k, 2l)**. These films included about 77 wt.% **(2a, 2d, 2g, 2j)**, about 74 wt.% **(2b, 2e, 2h, 2k)**, and about 66 wt.% **(2c, 2c, 2f, 2i, 2l)** PVOH resin, based on the total weight of the film. Films have a thickness of about 75 microns were prepared. The films were tested for cold water solubility at 10°C according to MSTM-205, for tensile strength according to the Tensile Strength Test, and for coefficient of friction according to the Coefficient of Friction Test. Films that dissolved in 100 seconds or less are indicated as (+) in Table 2 and films that dissolved in 300 seconds or less but more than 100 seconds are indicated as (*) in Table 2, below. Films that had a tensile strength of 45 MPa or greater are indicated as (+) in Table 2, films that had a tensile strength less than about 45 MPa but at least about 40 MPa or greater are indicated as (*) in Table 2, and films having a tensile strength of less than 40 MPa are indicated as (-) in Table 2, below. Films having a gloss-to-gloss static coefficient of friction of about 1 or less are indicated as (+) in Table 2, films having a gloss-to-gloss coefficient of friction about 5 or less, but greater than about 1 are indicated as (*) in Table 2, and films having a gloss-to-gloss coefficient of friction of greater than about 5 are indicated as (-) in Table 2, below.

| Table 2 Sample (phr starch) | Solubility | Tensile Strength (MPa) | Coefficient of Friction |
|---|---|---|---|
| 2a (2.66) | * | + (51.82) | - |
| 2b (7.98) | * | + (49.23) | * |
| 2c (23.94) | * | * (44.49) | * |
| 2d (2.66) | * | + (50.83) | + |
| 2e (7.98) | * | + (45.07) | + |
| 2f (23.94) | * | * (39.65) | + |
| 2g (2.66) | + | + (47.85) | + |
| 2h (7.98) | + | * (41.08) | + |
| 2i (23.94) | * | - (33.87) | + |
| 2j (2.66) | + | + (55.47) | - |
| 2k (7.98) | + | + (44.53) | + |
| 2l (23.94) | + | - (33.69) | + |

Table 2 shows that water-soluble films according to the disclosure including a water-soluble polyvinyl alcohol, plasticizer, and an unmodified starch having an amylose content in a range of about 20 wt.% to about 80 wt.% demonstrate good tensile strength and coefficients of friction when the starch is provided at a low loading level (e.g., about 2 phr to about 5 phr) **(2d, 2g)**. Table 2 further demonstrates that the tensile strength of a water-soluble films according to the disclosure including an unmodified starch having an amylose content in a range of about 20 wt.% to about 80 wt.% decreases with increasing starch loading. Thus, Table 2 demonstrates water-soluble films of the disclosure comprising a mixture of a water-soluble polyvinyl alcohol, plasticizer, and an unmodified starch having an amylose content in a range of about 20 wt.% and 80 wt.% and present in an amount of about 5 phr to about 30 phr that have good convertibility, and good film-to-film anti stick properties as demonstrated by a low G-G coefficient of friction.

Example 3

Water-soluble films having different polyvinyl alcohol resins were prepared and test for gloss-to-gloss static coefficient of friction. Each water-soluble film formulation included a polyvinyl alcohol resin, provided in an amount of about 74 wt.%, based on the total weight of the film. The polyvinyl alcohol resins tested included a PVOH homopolymer having a viscosity of about 13 cP and a degree of hydrolysis of about 88 (3a), a PVOH homopolymer having a viscosity of about 23 CP and a degree of hydrolysis of about 88 (3b), a polyvinyl alcohol copolymer having a 5% methyl acrylate modification (3c), a polyvinyl alcohol copolymer having a 4% maleate modification (3d), a polyvinyl alcohol copolymer having about a 2% maleate modification (3e), and a polyvinyl alcohol copolymer having about a 4% 2-acrylamido-2-methylpropane sulfonic acid modification. Each film formulation further included about 26 phr plasticizers, minor amounts of processing aids e.g., surfactants, antioxidants, etc. totaling about 1 phr, and a starch present at a level of about 7.98 phr. The starches tested included an unmodified starch having an amylose content of 50% **(3a[1]-3e[1])** or a hydroxypropyl modified starch having an amylose content of about 80% **(3a[2]-3e[2]).** Films having a gloss-to-gloss static coefficient of friction of about 1 or less are indicated as (+) in Table 3, films having a gloss-to-gloss coefficient of friction of about 5 or less but greater than about 1 are indicated as (*) in Table 3, and films having a gloss-to-gloss coefficient of friction of greater than about 5 are indicated as (-) in Table 3. Below.

| Table 3 | 3a[1] | 3b[1] | 3c[1] | 3d[1] | 3e[1] | 3f[1] |
|---|---|---|---|---|---|---|
| Sample COF | + | + | + | + | + | + |
| Sample | 3a[2] | 3b[2] | 3c[2] | 3d[2] | 3e[2] | 3f[2] |
| COF | + | * | - | * | + | * |

Table 3 shows that the effect of the unmodified starch on the gloss-to-gloss coefficient of friction was not dependent on the type of polyvinyl alcohol or the degree of modification of the polyvinyl alcohol copolymers. Table 3 further shows that the hydroxypropylated starch provided acceptable coefficient of frictions for all resin types except for the methyl acrylate modified PVOH. Thus, the effect of the hydroxypropylated starch is not significantly different across resin types, with the exception of acrylate modified PVOH.

**Example 4**

Water-soluble films having different starches were prepared and tested for tensile strength and tear strength according to the Tensile Strength Test and Tear Strength Test. Each water-soluble film formulation included a polyvinyl alcohol copolymer having maleate modification, provided in an amount of about 77 wt.%, based on the total weight of the film. The film formulations further included about 26 phr plasticizers, minor amounts of processing aids e.g., surfactants, antioxidants, etc. totaling about 1 phr, and a starch present at a level of about 2.66 phr. The starches tested included an unmodified starch having an amylose content of 1% **(4a),** an acid-hydrolyzed starch having an amylose content of 1% **(4b),** an unmodified starch having an amylose content of about 25% **(4c),** a hydroxyethyl modified starch having an amylose content of about 25% **(4d),** an unmodified starch having an amylose content of 50% **(4e),** an unmodified starch having an amylose content of 80% **(4f),** and a hydroxypropyl modified starch having an amylose content of 80% **(4g).** The tensile strength values and tear strength values are provided in Table 4, below.

| Table 4 | 4a | 4b | 4c | 4d | 4e | 4f | 4g |
|---|---|---|---|---|---|---|---|
| Sample Tensile Strength (N/mm$^2$) | 51.82 | 47.25 | 50.83 | 53.53 | 47.85 | 55.47 | 49.84 |
| Tear Strength (g/mil) | 1797.22 | 1984.6 | 1803.01 | 1954.97 | 1673.16 | 1442.5 | 1302.07 |

Table 4 shows that the tensile strength (about 45-55 N/mm$^2$) and tear strength (about 1300 to 2000 g/mil) of the water-soluble films are relatively consistent for all films. Accordingly, Table 4 demonstrates that the amylose content and modification type do not significantly impact the mechanical properties of the water-soluble films.

## Example 5

Water-soluble films having different polyvinyl alcohol to starch ratios were prepared and tested for tensile strength and tear strength as described above. Each water-soluble film formulation included a polyvinyl alcohol copolymer having maleate modification. The film formulations further included about 26 phr plasticizers, minor amounts of processing aids e.g., surfactants, antioxidants, etc. totaling about 1 phr, and an unmodified starch having an amylose content of about 50%. The amount of PVOH provided in the resin, based on the total weight of the film, and the ratio of PVOH:starch were 79 wt% PVOH and 100:0 (5a), 77 wt.% and 97:3 (5b), 63 wt.% and 80:20 (5c), 47 wt.% and 60:40 (5d), or 32 wt.% 40:60 (5e). Films that had a tensile strength of about 45 MPa or greater are indicated as (+) in Table 2, films that had a tensile strength of about 40 MPa or greater but less than about 45 MPa are indicated as (*) in Table 2, and films having a tensile strength of less than 40 MPa are indicated as (-) in Table 2, below. Films that had a tear strength of about 1500 g/mil or greater are indicated as (+) in Table 5, films that had a tensile strength of about 500 g/mil or greater but less than about 1500 g/mil are indicated as (*) in Table 5, and films having a tensile strength of less than 500 g/mil are indicated as (-) in Table 5, below.

| Table 5 | 5a | 5b | 5c | 5d | 5e |
|---|---|---|---|---|---|
| Sample Tensile strength | +(47.16) | + (51.06) | - (33.50) | - (23.28) | - (24.14) |
| (MPa) Tear strength (g/mil) | + (1774.61) | + (1844.24) | - (159.07) | - (0) | - (0) |

Table 5 shows that when an unmodified starch is present in a water-soluble film in a PVOH resin to starch ratio of about 80:20 or greater, the tensile strength and tear strength of the film dramatically decrease relative to that of an equivalent water-soluble film including starch in a PVOH resin to starch ratio of less than 80:20, e.g., 97:3.

## Examples 6 - 45

Water-soluble films having different polyvinyl alcohols present in an amount between about 20 wt.% and 95 wt.%, different starches present in an amount between about 2.5 phr and 30 phr, plasticizers present in an amount between about 10 wt.% to about 45 wt.%, surfactants present in an amount between about 0.1 wt.% and 8.0 wt.%, bittering agents present in an amount between about 0.01 wt.% and 10 wt.%, and bleaching agents present in an amount between about 0.01 wt.% and 10 wt.% are prepared. The film formulations are shown in Table 6, below.

| | Resin about 20 wt.% to about 95 wt.%, based on the total weight of the film | | | | | | | | | | | | | | | | Starch about 2.5 phr to about 30 phr | Plasticizer about 10 wt.% to about 45 wt.% based on the total weight of the film | | Surfactant about 0.1 wt% to about 8.0 wt% based on the total weight of the film | | 0.01 wt.% to 10 wt% based on the total weight of the film | | |
|---|---|---|---|---|---|---|---|---|---|---|---|---|---|---|---|---|---|---|---|---|---|---|---|---|
| | | | | | | | | | | | | | | | | | | Glycerine | Optionally one or more plasticizers* | Nonionic | Optionally one or more surfactants** | Bitrex ® | Sodium metabisulfite | Moisture content of the film |
| Ex | A | B | C | D | E | F | G | H | I | J | K | L | M | N | O | P | | | | | | | | |
| 1 | X | | | | | | | | | | | | | | X | | | X | X | X | X | X | X | 4-9 wt% |
| 2 | X | | | | | | | | | | | | | X | | | | X | X | X | X | X | X | 4-9 wt% |
| 3 | X | | | | | | | | | | | | | | | X | X | X | X | X | X | X | X | 4-9 wt% |
| 4 | X | | | X | | | | | | | | | | | X | | | X | X | X | X | X | X | 4-9 wt% |
| 5 | | X | | | | | | | | | | | | | X | | | X | X | X | X | X | X | 4-9 wt% |

| No. | | | | | | | | | | | | | | | | | | | | | | |
|---|---|---|---|---|---|---|---|---|---|---|---|---|---|---|---|---|---|---|---|---|---|---|
| 6 | | | X | | | | | | | | | | | | X | | X | X | X | X | X | 4-9 wt% |
| 7 | | | X | X | | | | | | | | | | | X | | X | X | X | X | X | 4-9 wt% |
| 8 | | | | | X | | | | | | | | | | X | | X | X | X | X | X | 4-9 wt% |
| 9 | | | | | | X | | | | | | | | | X | | X | X | X | X | X | 4-9 wt% |
| 10 | | | | | | | X | | | | | | | | X | | X | X | X | X | X | 4-9 wt% |
| 11 | | | | | | | | | X | | | | | | X | | X | X | X | X | X | 4-9 wt% |
| 12 | | | | | | | | | | X | | | | | X | | X | X | X | X | X | 4-9 wt% |
| 13 | | | | | | | | | | | X | | | | X | | X | X | X | X | X | 4-9 wt% |
| 14 | | | | | | | | | | | | X | | | X | | X | X | X | X | X | 4-9 wt% |
| 15 | | | | | | | | | | | | | X | | X | | X | X | X | X | X | 4-9 wt% |
| 16 | | | | | | | | | | | | | | X | X | | X | X | X | X | X | 4-9 wt% |
| 17 | | | X | | | | | | | | X | | | | X | | X | X | X | X | X | 4-9 wt% |
| 18 | | | X | | | | | | | | | X | | | X | | X | X | X | X | X | 4-9 wt% |
| 19 | | | X | | | | | | | | | | X | | X | | X | X | X | X | X | 4-9 wt% |

| No. | | | | | | | | | | | | | | | | | | | | | | |
|---|---|---|---|---|---|---|---|---|---|---|---|---|---|---|---|---|---|---|---|---|---|---|
| 20 | | | X | | | | | | | | | | | X | X | | X | X | X | X | X | 4-9 wt% |
| 21 | | | X | | X | | | | | | X | | | | X | | X | X | X | X | X | 4-9 wt% |
| 22 | | | X | | X | | | | | | | | X | | X | | X | X | X | X | X | 4-9 wt% |
| 23 | | | X | | X | | | | | | | | | X | X | | X | X | X | X | X | 4-9 wt% |
| 24 | X | | | | | | | | | | | | | | X | | X | X | X | X | X | 4-9 wt% |
| 25 | | X | | | | | | | | | | | | | X | | X | X | X | X | X | 4-9 wt% |
| 26 | | | X | | | | | | | | | | | | X | | X | X | X | X | X | 4-9 wt% |
| 27 | | | | X | | | | | | | | | | | X | | X | X | X | X | X | 4-9 wt% |
| 28 | | | | | X | | | | | | | | | | | X | | X | X | X | X | 4-9 wt% |
| 29 | | | | | | X | | | | | | | | | | X | | X | X | X | X | 4-9 wt% |
| 30 | | | | | | | X | | | | | | | | | X | | X | X | X | X | 4-9 wt% |
| 31 | | | | | | | | | X | | | | | | | X | | X | X | X | X | 4-9 wt% |
| 32 | | | | | | | | | | | X | | | | | X | | X | X | X | X | 4-9 wt% |
| 33 | | | | | | | | | | | | X | | | | X | | X | X | X | X | 4-9 wt% |

| Ex. | | | | | | | | | | | | | | | | | | | | |
|---|---|---|---|---|---|---|---|---|---|---|---|---|---|---|---|---|---|---|---|---|
| 34 | | | | | | | | | X | | | X | X | X | X | X | X | X | | 4-9 wt% |
| 35 | | | | | | | | | | X | | X | X | X | X | X | X | X | | 4-9 wt% |
| 36 | | | | | | | | | | | X | X | X | X | X | X | X | X | | 4-9 wt% |
| 37 | X | | | X | | | | | | | | X | X | X | X | X | X | X | | 4-9 wt% |
| 38 | | X | X | | | | | | | | | X | X | X | X | X | X | X | | 4-9 wt% |
| 39 | | | X | | X | | | | X | | | X | X | X | X | X | X | X | | 4-9 wt% |
| 40 | | X | | | | | | | | | X | X | | X | X | X | X | X | X | 4-9 wt% |

\* selected from one or more of the following: polyethylene glycol, sorbitol, trimethylolpropane, 2-methyl-1,3-propanediol, dulcitol, erythritol, glycerol propylene oxide polymers, hexylene glycol, propylene glycol, triethylene glycol, voranol, xylitol

\*\* selected as one or more of the following: cationic, anionic, zwitterionic

A is a PVOH terpolymer of alkyl acrylate monomer

B is a PVOH terpolymer of 2-acrylamide-2-methylpropanesulfonic acid monomer unit

C is a PVOH terpolymer of monoalkyl maleate monomer unit at 1.75 mole %

D is a PVOH terpolymer of monoalkyl maleate monomer unit at 4.00 mole %

E is a PVOH homopolymer (viscosity 3; DH 85)

F is a PVOH homopolymer (viscosity 4; DH 88)

G is a PVOH homopolymer (viscosity 8; DH 88)

H is a PVOH homopolymer (viscosity 13; DH 88)

I is a PVOH homopolymer (viscosity 15; DH 79)

J is a PVOH homopolymer (viscosity 23; DH 88)

K is a PVOH homopolymer (viscosity 32; DH 88)

L is a PVOH homopolymer (viscosity 40; DH 88)

M is a PVOH homopolymer (viscosity 56; DH 98)

N is Casco Corn Products (25% Amylose, Native)(Ingredion)

O is Hylon® V (50% Amylose, Native) (Ingredion)

P is Eco-Film® (80% Amylose, Hydroxypropylated, ~2.5% mod) (Ingredion)

Thus, Examples 6 to 45 show film formulations specifically contemplated for preparation of water soluble films according to the disclosure.

## Example 46

Pouches are prepared from the water-soluble films of Examples 1 to 45. In particular, pouches are formed from two layers of water-soluble polymer film sealed at an interface, or by a single film that is folded upon itself and sealed. In both methods, the film defines an interior pouch container that is sealed at a matte-to-matte interface, with the gloss side of the film forming the exterior of the pouch.

The pouches prepared are further filled with compositions comprising liquids, powders, gels, or combinations thereof. The compositions comprise components such as surfactants, bleaches, enzymes, perfumes, dyes or colorants, and/or solvents (for liquid and gel compositions).

## Example 47

A water-soluble film according to the disclosure was thermoformed into filled pouches using commercial, high-speed equipment. The pouches demonstrated the practical, real-world consequence of lowering the coefficient of friction of the water-soluble film. In particular, while commercial films having a similar composition but not including the starch of the disclosure require application of a powder (e.g. talc, as described in U.S. Patent No. 9,290,727) to prevent the formed pouches from sticking together during handling, pouches made from a film according to the invention did not require powder application in order to prevent pouch-to-pouch sticking. The observed benefit was a result of the film of the disclosure having a reduced coefficient of friction.

## Claims

1. A water-soluble film, comprising a mixture of a water-soluble polyvinyl alcohol, a plasticizer, and a hydroxypropyl-modified starch, wherein the polyvinyl alcohol is present in an amount of 20 wt.% to 95 wt.%, based on the total weight of the film; and wherein the hydroxypropyl-modified starch:

(a) has an amylose content in a range of 65% to 95% by weight, based on the total weight of amylose and amylopectin in the hydroxypropyl-modified starch, and is present in an amount in a range of 5 phr to 30 phr;
(b) has a degree of modification of greater than 2% by weight, has an amylose content in a range of 65% to 95% by weight, based on the total weight of amylose and amylopectin in the hydroxypropyl-modified starch, and is present in an amount in a range of 2.5 phr to 30 phr; or
(c) has an amylose content in a range of 23 to 95% by weight, based on the total weight of amylose and amylopectin in the hydroxypropyl-modified starch, and is present in an amount in a range of 5 phr to 30 phr, wherein the polyvinyl alcohol comprises an unmodified polyvinyl alcohol or an anionic modified polyvinyl alcohol with the proviso that the anionic modifier is not an acrylate,

wherein phr denotes parts per one hundred parts of the total water-soluble polyvinyl alcohol content of the film.

2. The water-soluble film of claim **1,** wherein, for alternative (a) or alternative (b), the hydroxypropyl-modified starch is present in an amount of 8 phr and is cold water soluble as **characterized by** a 76 micron film having a cold water solubility at 10 °C of less than 90 seconds as measured by MSTM-205.

3. The water-soluble film of any one of the preceding claims, wherein the film has a tensile strength of at least 40 MPa, or in a range of 45 MPa to 60 MPa, as measured according to ASTM D882.

4. The water-soluble film of any one of the preceding claims, wherein the film has a gloss to gloss static coefficient of friction of 5 or less, as measured according to ASTM D1894.

5. The water-soluble film of any one of the preceding claims, wherein, for alternative (a) or alternative (b), the polyvinyl alcohol comprises a polyvinyl alcohol homopolymer, a polyvinyl alcohol copolymer having an anionic modification, or a combination of the foregoing.

6. The water-soluble film of any one of the preceding claims, wherein, for alternative (b), the polyvinyl alcohol comprises a polyvinyl alcohol copolymer having an anionic modification.

7. The water-soluble film of any one of the preceding claims, wherein, for alternative (c), the polyvinyl alcohol is present in an amount of 60 wt.% to 95 wt.%, or 85 to 95 wt.%, based on the total weight of the film.

8. The water-soluble film of any one of the preceding claims, wherein the hydroxypropyl-modified starch comprises greater than 2% modification by weight.

9. The water-soluble film of any one of the preceding claims, wherein the hydroxypropyl-modified starch has a degree of modification of 4% to 8% by weight.

10. The water-soluble film of any one of the preceding claims, wherein, for alternative (c), the hydroxypropyl-modified starch has an amylose content in a range of 50% to 95%, or 65% to 95%, by weight, based on the total weight of amylose and amylopectin in the hydroxypropyl-modified starch.

11. The water-soluble film of any one of the preceding claims 1 or 3 to 10 when not depending on claim 2, wherein the hydroxypropyl-modified starch is present in an amount in a range of 5 phr to 12 phr, 6 phr to 10 phr, or 12 phr to 16 phr.

12. The water-soluble film of any one of the preceding claims, wherein the plasticizer is present in an amount in a range of 1 phr to 40 phr.

13. The water-soluble film of any one of the preceding claims, wherein the plasticizer is selected form the group consisting of glycerol, propylene glycol, sorbitol, trimethylolpropane, polyethylene glycols, glycerol propylene oxide polymers, 2-methyl-1,3-propanediol, diglycerol, xylitol, and combinations of the foregoing.

14. The water-soluble film of any one of the preceding claims, wherein the plasticizer is selected from the group consisting of 2-methyl-1,3-propanediol, sorbitol, glycerol, and combinations thereof.

15. The water-soluble film of any one of the preceding claims, further comprising a surfactant, an antioxidant, a bittering agent, a soil release polymer, an anti-redeposition aid, a chelant, a builder, a perfume, or a combination of the foregoing.

16. The water-soluble film of any one of the preceding claims, wherein the film is **characterized by** a reduced gloss to gloss static coefficient of friction as **characterized by** the gloss to gloss static coefficient of friction of the film being reduced compared to the equivalent film formulation that does not include a starch.

17. A sealed article comprising the water-soluble film of any one of the preceding claims in the form of a pouch defining an interior pouch volume.

18. The sealed article of claim 17, further comprising a detergent composition contained in the interior pouch volume, wherein the detergent composition is selected from the group consisting of a laundry detergent, a dishwashing detergent, a hard surface cleaning composition, a fabric enhancer composition, a shampoo, a body wash, and combinations thereof.


**Patentansprüche**

1. Wasserlösliche Folie, die ein Gemisch aus einem wasserlöslichen Polyvinylalkohol, einem Weichmacher und einer hydroxypropylmodifizierten Stärke aufweist, wobei der Polyvinylalkohol in einer Menge von 20 Gew.-% bis 95 Gew.-%, bezogen auf das Gesamtgewicht der Folie, vorhanden ist; und wobei die hydroxypropylmodifizierte Stärke:

    (a) einen Amylosegehalt in einem Bereich von 65 bis 95 Gew.-%, bezogen auf das Gesamtgewicht von Amylose und Amylopektin in der hydroxypropylmodifizierten Stärke, aufweist und in einer Menge in einem Bereich von 5 phr bis 30 phr vorhanden ist;
    (b) einen Modifizierungsgrad von mehr als 2 Gew.-% aufweist, einen Amylosegehalt im Bereich von 65 bis 95 Gew.-%, bezogen auf das Gesamtgewicht von Amylose und Amylopektin in der hydroxypropylmodifizierten Stärke, hat und in einer Menge im Bereich von 2,5 phr bis 30 phr vorhanden ist; oder
    (c) einen Amylosegehalt im Bereich von 23 bis 95 Gew.-%, bezogen auf das Gesamtgewicht von Amylose und Amylopektin in der hydroxypropylmodifizierten Stärke, aufweist und in einer Menge im Bereich von 5 phr bis 30 phr vorhanden ist, wobei der Polyvinylalkohol einen unmodifizierten Polyvinylalkohol oder einen anionisch modifizierten Polyvinylalkohol aufweist, mit der Maßgabe, dass der anionische Modifikator kein Acrylat ist,

    wobei phr Teile pro hundert Teile des gesamten wasserlöslichen Polyvinylalkoholgehalts der Folie bezeichnet.

2. Wasserlösliche Folie nach Anspruch 1, wobei bei Alternative (a) oder Alternative (b) die hydroxypropylmodifizierte Stärke in einer Menge von 8 phr vorhanden ist und in kaltem Wasser löslich ist, **gekennzeichnet durch** eine 76-Mikrometer-Folie mit einer Kaltwasserlöslichkeit bei 10 °C von weniger als 90 Sekunden, gemessen gemäß MSTM-205.

3. Wasserlösliche Folie nach einem der vorhergehenden Ansprüche, wobei die Folie eine Zugfestigkeit von mindestens 40 MPa oder in einem Bereich von 45 MPa bis 60 MPa, gemessen gemäß ASTMD882, aufweist.

4. Wasserlösliche Folie nach einem der vorhergehenden Ansprüche, wobei die Folie einen statischen Glanz-Glanz-Reibungskoeffizienten von 5 oder weniger, gemessen gemäß ASTM D1894 aufweist.

5. Wasserlösliche Folie nach einem der vorangehenden Ansprüche, wobei der Polyvinylalkohol für Alternative (a) oder Alternative (b) ein Polyvinylalkohol-Homopolymer, ein Polyvinylalkohol-Copolymer mit einer anionischen Modifikation oder eine Kombination der vorgenannten aufweist.

6. Wasserlösliche Folie nach einem der vorhergehenden Ansprüche, wobei für Alternative (b) der Polyvinylalkohol ein Polyvinylalkohol-Copolymer mit einer anionischen Modifikation aufweist.

7. Wasserlösliche Folie nach einem der vorhergehenden Ansprüche, wobei bei Alternative (c) der Polyvinylalkohol in einer Menge von 60 Gew.-% bis 95 Gew.-% oder 85 bis 95 Gew.-%, bezogen auf das Gesamtgewicht der Folie, vorhanden ist.

8. Wasserlösliche Folie nach einem der vorhergehenden Ansprüche, wobei die hydroxypropylmodifizierte Stärke mehr als 2 Gew.-% Modifikation aufweist.

9. Wasserlösliche Folie nach einem der vorhergehenden Ansprüche, wobei die hydroxypropylmodifizierte Stärke einen Modifizierungsgrad von 4 bis 8 Gew.-% aufweist.

10. Wasserlösliche Folie nach einem der vorhergehenden Ansprüche, wobei für Alternative (c) die hydroxypropylmodifizierte Stärke einen Amylosegehalt im Bereich von 50 bis 95 Gew.-% oder 65 bis 95 Gew.-%, bezogen auf das Gesamtgewicht von Amylose und Amylopektin in der hydroxypropylmodifizierten Stärke, aufweist.

11. Wasserlösliche Folie nach einem der vorhergehenden Ansprüche 1 oder 3 bis 10, wenn nicht von Anspruch 2 abhängig, wobei die hydroxypropylmodifizierte Stärke in einer Menge im Bereich von 5 phr bis 12 phr, 6 phr bis 10 phr oder 12 phr bis 16 phr vorhanden ist.

12. Wasserlösliche Folie nach einem der vorhergehenden Ansprüche, wobei der Weichmacher in einer Menge im Bereich von 1 phr bis 40 phr vorhanden ist.

13. Wasserlösliche Folie nach einem der vorhergehenden Ansprüche, wobei der Weichmacher aus der Gruppe ausgewählt ist, die aus Glycerin, Propylenglycol, Sorbit, Trimethylolpropan, Polyethylenglycolen, Glycerin-Propylenoxid-Polymeren, 2-Methyl-1,3-Propandiol, Diglycerin, Xylit und Kombinationen der vorgenannten besteht.

14. Wasserlösliche Folie nach einem der vorhergehenden Ansprüche, wobei der Weichmacher Gruppe ausgewählt ist, die aus 2-Methyl-1,3-Propandiol, Sorbit, Glycerin und Kombinationen davon besteht.

15. Wasserlösliche Folie nach einem der vorhergehenden Ansprüche, die ferner ein Tensid, ein Antioxidationsmittel, einen Bitterstoff, ein Polymer zur Schmutzablösung, ein Mittel zur Verhinderung der Wiederablagerung, einen Chelatbildner, einen Gerüststoff, einen Duftstoff oder eine Kombination der vorgenannten Stoffe aufweist.

16. Wasserlösliche Folie nach einem der vorhergehenden Ansprüche, wobei die Folie durch einen verringerten statischen Glanz-Glanz-Reibungskoeffizienten **gekennzeichnet ist, dadurch** gekennzeichnet, dass der statische Glanz-Glanz-Reibungskoeffizienten der Folie im Vergleich zu einer äquivalenten Folienformulierung, die keine Stärke aufweist, verringert ist.

17. Versiegelter Artikel, der die wasserlösliche Folie nach einem der vorhergehenden Ansprüche in Form eines Beutels aufweist, der ein inneres Beutelvolumen definiert.

18. Versiegelter Artikel nach Anspruch 17, der ferner eine in dem inneren Beutelvolumen enthaltene Waschmittelzusammensetzung aufweist, wobei die Waschmittelzusammensetzung aus der Gruppe ausgewählt ist, die aus einem Waschmittel, einem Geschirrspülmittel, einer Reinigungszusammensetzung für harte Oberflächen, einer Textilerstärkungszusammensetzung, einem Shampoo, einem Körperwaschmittel und Kombinationen davon besteht.


**Revendications**

1. Film hydrosoluble, comprenant un mélange d'un alcool polyvinylique hydrosoluble, d'un plastifiant et d'un amidon hydroxypropylé, où l'alcool polyvinylique est présenté dans une proportion de 20 % en poids à 95 % en poids par rapport au poids total du film ; et où l'amidon hydroxypropylé :

   (a) a une teneur en amylose comprise entre 65 % et 95 % en poids, sur la base du poids total d'amylose et d'amylopectine dans l'amidon hydroxypropylé, et est présenté dans une quantité comprise entre 5 pce et 30 pce ;
   (b) a un degré de modification supérieur à 2 % en poids, une teneur en amylose comprise entre 65 % et 95 % en poids, sur la base du poids total d'amylose et d'amylopectine dans l'amidon hydroxypropylé, et est présenté dans une quantité comprise entre 2,5 pce et 30 pce ; ou
   (c) a une teneur en amylose comprise entre 23 et 95 % en poids, sur la base du poids total d'amylose et d'amylopectine dans l'amidon hydroxypropylé, et est présenté dans une quantité comprise entre 5 et 30 pce, l'alcool polyvinylique comprenant un alcool polyvinylique non modifié ou un alcool polyvinylique modifié anionique, à condition que le modificateur anionique ne soit pas un acrylate,

où pce désigne les parties par centaine de la teneur totale en alcool polyvinylique hydrosoluble du film.

2. Film hydrosoluble selon la revendication 1, où, pour la variante (a) ou la variante (b), l'amidon hydroxypropylé est présenté dans une quantité de 8 pce et est soluble dans l'eau froide, **caractérisé par** un film de 76 microns ayant une solubilité inférieure à 90 secondes dans l'eau froide à 10 °C, mesurée conformément à la méthode MSTM-205.

3. Film hydrosoluble selon l'une des revendications précédentes, où ledit film présente une résistance à la traction d'au moins 40 MPa, ou comprise entre 45 MPa et 60 MPa, mesurée conformément à la norme ASTM D882.

4. Film hydrosoluble selon l'une des revendications précédentes, où ledit film a un coefficient de frottement statique de brillant à brillant égal ou inférieur à 5, mesuré conformément à la norme ASTM D1894.

5. Film hydrosoluble selon l'une des revendications précédentes, où, pour la variante (a) ou la variante (b), l'alcool polyvinylique comprend un homopolymère d'alcool polyvinylique, un copolymère d'alcool polyvinylique ayant une modification anionique, ou une combinaison de ceux-ci.

6. Film hydrosoluble selon l'une des revendications précédentes, où, pour la variante (b), l'alcool polyvinylique comprend un copolymère d'alcool polyvinylique ayant une modification anionique.

7. Film hydrosoluble selon l'une des revendications précédentes, où, pour la variante (c), l'alcool polyvinylique est présenté dans une quantité de 60 % en poids à 95 % en poids, ou de 85 à 95 % en poids, par rapport au poids total du film.

8. Film hydrosoluble selon l'une des revendications précédentes, où l'amidon hydroxypropylé comprend plus de 2 % de modification en poids.

9. Film hydrosoluble selon l'une des revendications précédentes, où l'amidon hydroxypropylé a un degré de modification de 4 % à 8 % en poids.

10. Film hydrosoluble selon l'une des revendications précédentes, où, pour la variante (c), l'amidon hydroxypropylé a une teneur en amylose comprise entre 50 % et 95 %, ou entre 65 % et 95 %, en poids, sur la base du poids total d'amylose et d'amylopectine dans l'amidon hydroxypropylé.

11. Film hydrosoluble selon l'une des revendications 1, ou 3 à 10, si non dépendante de la revendication 2, où l'amidon hydroxypropylé est présenté dans une quantité comprise entre 5 pce et 12 pce, 6 pce et 10 pce, ou 12 pce et 16 pce.

12. Film hydrosoluble selon l'une des revendications précédentes, où le plastifiant est présenté dans une quantité comprise entre 1 et 40 pce.

13. Film hydrosoluble selon l'une des revendications précédentes, où le plastifiant est sélectionné dans le groupe constitué par le glycérol, le propylène glycol, le sorbitol, le triméthylolpropane, les polyéthylène glycols, les polymères d'oxyde de glycérol-propylène, le 2-méthyl-1,3-propanediol, le diglycérol, le xylitol, et des combinaisons de ceux-ci.

14. Film hydrosoluble selon l'une des revendications précédentes, où le plastifiant est sélectionné dans le groupe constitué par le 2-méthyl-1,3-propanediol, le sorbitol, le glycérol, et des combinaisons de ceux-ci.

15. Film hydrosoluble selon l'une des revendications précédentes, comprenant en outre un tensioactif, un antioxydant, un agent d'amertume, un polymère antisalissure, un agent anti-redéposition, un agent de chélation, un adjuvant, un parfum, ou une combinaison de ceux-ci.

16. Film hydrosoluble selon l'une des revendications précédentes, où ledit film est **caractérisé par** un coefficient de frottement statique de brillant à brillant réduit par rapport à la formulation de film équivalente ne comprenant pas d'amidon.

17. Article scellé comprenant le film hydrosoluble selon l'une des revendications précédentes sous la forme d'un sachet définissant un volume intérieur du sachet.

18. Article scellé selon la revendication 17, comprenant en outre une composition détergente contenue dans le volume intérieur du sachet, ladite composition détergente étant sélectionnée dans le groupe constitué d'un détergent pour le linge, d'un détergent pour la vaisselle, d'une composition pour le nettoyage des surfaces dures, d'une composition assouplissante pour textiles, d'un shampoing, d'un gal douche, et de combinaisons de ceux-ci.

Figure 1

18  Upper Grip

20  Pull Cord

22  Pulley

10  Friction Fixture

12  Friction Sled

Lower
Coupling 24

Film 14

Figure 2

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2004074351 A2
- US 2016280869 A1
- GB 2442626 A
- EP 0611804 A1
- US 5300145 A
- EP 08101442
- US 2013240388 A1
- WO 2009152031 A
- US 8835372 B
- US 6204223 B
- US 4681228 A
- EP 0989803 A1
- US 5558228 A
- US 8333033 B
- US 20140110301
- US 8728593 B
- US 7476325 B
- US 20080185347
- US 20140124454
- US 20060172910 A
- US RE29059 E
- US 20040144682
- US 20060173430 A
- US 3580390 A
- US 20110054111
- US 8163104 B
- US 20070003719
- US 4466431 A
- US 7022656 B
- US 9290727 B

## Non-patent literature cited in the description

- Effect of starch type on the properties of starch containing films. **LAWTON J. W.** CARBOHYDRATE POLYMERS. APPLIED SCIENCE PUBLISHERS, 01 March 1996, vol. 29, 203-208
- **F.L. BATES ; D. FRENCH ; R.E. RUNDLE**. *J. Am. Chem. Soc.*, 1943, vol. 65 (2), 142-148
- **SHI, Y. ; CAPITANI, T. ; TRZASKO, P. ; JEFFOAT, R.** *Journal of Cereal Science*, 1998, vol. 27, 289-299